# EUROPÄISCHE PATENTSCHRIFT

(11) **EP 0 376 156 B1**
(45) Veröffentlichungstag und Bekanntmachung des Hinweises auf die Patenterteilung: **27.03.1996**
(21) Anmeldenummer: 89123616.8
(22) Anmeldetag: 21.12.1989
(51) Int. Cl.: C07K 14/62, A61K 38/28, C12P 21/02

(54) **Neue Insulinderivate, Verfahren zu deren Herstellung, ihre Verwendung und eine sie enthaltende pharmazeutische Zubereitung**
New insulin derivatives, process for their preparation, their use and pharmaceutical compositions containing them
Nouveaux dérivés d'insuline, procédé de leur fabrication, leur utilisation et composés pharmaceutiques les contenant

(30) Priorität: 29.12.1988 DE 3844211
(43) Veröffentlichungstag der Anmeldung: 04.07.1990
(73) Patentinhaber: HOECHST AKTIENGESELLSCHAFT, D-65926 Frankfurt am Main (DE)
(72) Erfinder: Vértesy, László, Dr., D-6239 Eppstein/Taunus (DE); Geisen, Karl, Dr., D-6000 Frankfurt am Main (DE); Riess, Günther Johannes, Dr., D-6230 Frankfurt am Main 80 (DE); Sauber, Klaus, Dr., D-6232 Bad Soden am Taunus (DE)

(56) Entgegenhaltungen:
- EP-A- 0 195 691
- PROC. NATL. ACAD. SCI. USA, Band 83, 1986, Seiten 6766-6770, Washington, US; L. THIM et al.: "Secretion and processing of insulin precursors in yeast"
- BIOCHEM. JOURNAL, Band 186, 1980, Seiten 945-952, Oxford, GB; P. RÖSEN et al.:"Binding of insulin to bovine liver plasma membrane"

## Beschreibung

Insulin und Insulinderivate werden bekanntlich in erheblichen Mengen zur Behandlung der Krankheit Diabetes mellitus benötigt und z.T. auch großtechnisch produziert. Trotz der beträchtlichen Zahl der bereits existierenden Insulin-Zubereitungen und -Abwandlungen mit unterschiedlichen Wirkungsprofilen besteht wegen der Verschiedenheit der Organismen mit deren inter- und intraindividuellen Schwankungen immer noch ein Bedarf an weiteren Insulinprodukten mit wieder anderen Eigenschaften und Wirkungscharakteristiken.

In den Dokumenten EP 0 195 691 und Proc. Natl. Acad. Sci. USA 83 (1986), 6766-6770 werden Verfahren beschrieben, die in hoher Ausbeute Humaninsulin ergeben. Als Zwischenprodukte treten an der B-Kette von Insulin verlängerte Insulinderivate auf.

Insulinderivate mit einer verzögerten Wirkung sind z.B. beschrieben in EP-B-132 769 und EP-B-132 770. Es handelt sich um speziell in der Position B31 der Insulin-B-Kette basisch modifizierte Derivate der folgenden Formel I: 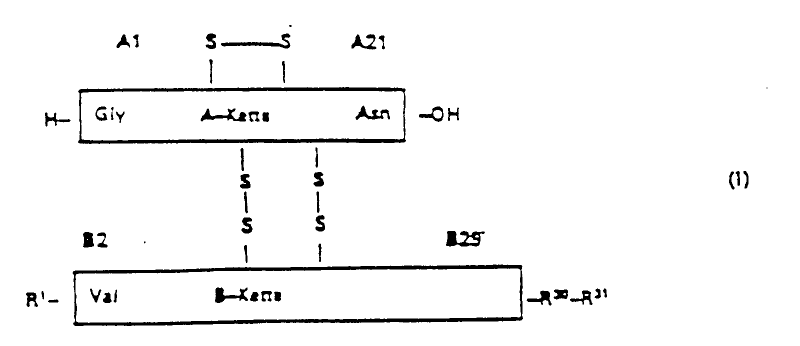
in welcher R¹ oder H-Phe bedeutet,
R³⁰ für den Rest einer neutralen, genetisch kodierbaren L-Aminosäure steht und
R³¹ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene endständige Carboxylfunktion frei, als Esterfunktion, als Amidfunktion, als Lacton oder zu CH₂OH reduziert vorliegen kann.

Für diese Insulinderivate ist ein isoelektrischer Punkt zwischen 5,8 und 8,5 (gemessen in der isoelektrischen Fokussierung) charakteristisch. Der - gegenüber dem isoelektrischen Punkt des unmodifizierten nativen Insulins oder Proinsulins (bei pH = 5,4) in den Neutralbereich hinein verschobene - isoelektrische Punkt ist durch die zusätzliche(n), an der Oberfläche des Moleküls befindliche(n) positive(n) Ladung(en) infolge der basischen Modifikation bedingt. Damit sind diese basisch modifizierten Insulinderivate im physiologisch wichtigen Neutralbereich weniger löslich als etwa natives Insulin oder Proinsulin, welche im Neutralbereich normalerweise gelöst vorliegen.

Die Verzögerungs- oder Depot-wirkung der basisch modifizierten Insulinderivate der Formel I geht auf deren Schwerlöslichkeit am isoelektrischen Punkt zurück. Die Wiederauflösung der Insulinderivate unter physiologischen Bedingungen soll nach den beiden vorerwähnten Druckschriften durch Abspaltung der zusätzlichen basischen Gruppen erreicht werden, was je nach Derivat durch tryptische oder Trypsin-ähnliche und/oder Carboxypeptidase B oder der Carboxypeptidase B-ähnliche und/oder Esterase-Aktivität zustande kommt. Die jeweils abgespaltenen Gruppen sind entweder rein physiologische Metaboliten oder aber leicht metabolisierbare, physiologisch unbedenkliche Substanzen.

Das vorerwähnte Depotprinzip infolge basischer Modifikation des Insulins wurde in der Folgezeit weiter genutzt durch die Bereitstellung und entsprechende Verwendung anderer - hauptsächlich innerhalb der A- und B-Ketten - basisch modifizierter Insulinderivate; vgl. z.B. EP-A-0194 864 und EP-A-0254 516.

Es sind auch einige basisch modifizierte Insulinderivate bekannt, bei denen sich die basische Modifizierung in der Verlängerung der A-Kette über die Position A1 hinaus befindet; vgl. P. Rösen et al., Biochem. J. (1980) 186, 945-952. Als solche Insulinderivate mit basischen Aminosäuren als Modifizierungskomponente sind in dieser Literaturstelle speziell beschrieben:

Lys-Arg-Gly^A1-Rinderinsulin
Arg-Gly^A1-Rinderinsulin
Arg-Arg-Gly^A1-Rinderinsulin und
Arg-Arg-Arg-Gly^A1-Rinderinsulin.

Diese Insulinderivate sollen gegenüber unmodifiziertem Insulin eine erheblich verringerte biologische Aktivität besitzen; vgl. insbesondere Tabelle 1 auf Seite 947 der genannten Literaturstelle. Über eine etwaige Depot-Wirkung der Verbindungen ist in der Literaturstelle nichts ausgesagt.

In dem Bestreben, das vorerwähnte Depot-Prinzip für die Behandlung der Krankheit Diabetes mellitus in vorteilhafter Weise weiter auszubauen und nutzbar zu machen, wurde nun eine neue Gruppe basisch modifizierter Insulinderivate gefunden; es handelt sich um im wesentlichen gereinigte Insulinderivate der nachstehenden Formel II, an deren AO-Position die basische Aminosäure Arginin sitzt: 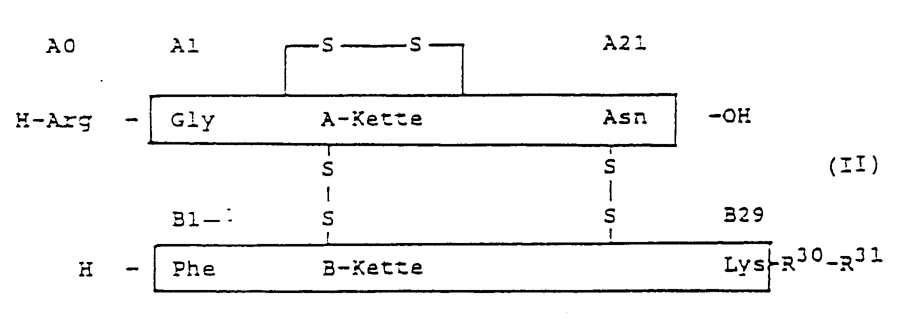
in welcher
a)
   - R³⁰ + R³¹: zusammen OH bedeuten oder
b)
   - R³⁰: bedeutet Rest einer neutralen, genetisch kodierbaren L-Aminosäure und
   - R³¹: bedeutet OH oder 1 bis 3 L-Aminosäuren,
mit Ausnahme des Falles, in welchem gleichzeitig R³⁰ = Ala, R³¹ = OH sowie die A- und B-Kette die Sequenzen des Rinderinsulins sind [d.i. also AO-Arg-Rinderinsulin].

Die physiologisch verträglichen Salze (wie z.B. die Alkali- oder Ammoniumsalze) dieser Insulinderivate sind in der Erfindung mit eingeschlossen.

Die neuen Insulinderivate weisen infolge ihrer basischen Modifikation in A0-Position - wie auch die bekannten basisch modifizierten Insulinderivate - ein verzögertes Wirkungsprofil und - gegenüber den bekannten basisch modifizierten Insulinderivaten - deutliche Vorteile in Bezug auf die Verträglichkeit im Organismus auf; außerdem ist im Hinblick auf die Literaturstelle P. Rösen et al. (a.a.O.) außerordentlich überraschend, daß ihre biologische Aktivität derjenigen von nativem Insulin entspricht.
a) Die Verbindungen der Formel II mit R³⁰ + R³¹ zusammen = OH sind die entsprechenden A0-Arg-Des-B30-Insuline; diese Verbindungen sind besonders bevorzugt.
b) Alternativ kann in Formel II R³⁰ auch der Rest einer neutralen, genetisch kodierbaren L-Aminosäure und
   R³¹ = OH oder
   eine entsprechende physiologisch unbedenkliche organische Gruppe basischen Charakters mit bis zu 50 C-Atomen sein.

Neutrale, genetisch kodierbare L-Aminosäuren - für R³⁰ - sind Gly, Ala, Ser, Thr, Val, Leu, Ile, Asn, Gln, Cys, Met, Tyr, Phe und Pro; bevorzugt sind Ala, Thr und Ser, insbesondere nur Thr.

Falls R³¹ = OH, resultieren Insulinderivate, welche sich von den entsprechenden Insulinen nur durch die Modifikation in der A0-Position (Arg) unterscheiden - im Falle der besonders bevorzugten neutralen genetisch kodierbaren L-Aminosäure Thr (und der A- sowie B1- bis B29-Kette = Sequenzen des Humaninsulins) ist dies A0-Arg-Humaninsulin.

Wenn am Aufbau von R³¹ bis zu 3 α-Aminosäuren beteiligt sind, sind dies in erster Linie neutrale oder basische natürlich vorkommende L-Aminosäuren und/oder die diesen entsprechenden D-Aminosäuren. Neutrale natürlich vorkommende Aminosäuren sind insbesondere Gly, Ala, Ser, Thr, Val, Leu, Ile, Asn, Gln, Cys, Met, Tyr, Phe, Pro und Hyp. Basische natürlich vorkommende Aminosäuren sind insbesondere Arg, Lys, Hyl, Orn, Cit und His.

Bevorzugt besteht R³¹ aus 1, 2 oder 3 der vorerwähnten basischen natürlich vorkommenden Aminosäuren; besonders bevorzugt ist R³¹ = Arg-OH oder Arg-Arg-OH.

Die besonders bevorzugte Bedeutung von R³¹ ist bevorzugt kombiniert mit R³⁰ = Ala, Thr oder Ser, insbesondere nur Thr. Mit R³⁰ = Thr und der A-kette und B1- bis B29-Kette = Sequenzen des Humaninsulins resultieren A0-Arg-B31-Arg-OH-Humaninsulin und A0-Arg-B31-Arg-B32-Arg-OH-Humaninsulin.

Die A-Kette und die B1- bis B29-Kette in Formel II können im Prinzip die Sequenzen aller möglichen Insuline sein; vorzugsweise sind sie jedoch die Sequenzen des Human-, Schweine- oder Rinder-Insulins, insbesondere die Sequenzen des Humaninsulins (welche mit den A1- bis A21- und B1- bis B29-Sequenzen des Schweineinsulins identisch sind).

Der isoelektrische Punkt der Insulinderivate der Formel II liegt zwischen 5,5 und 9,0 (gemessen in der isoelektrischen Fokussierung).

Die Herstellung der Insulinderivate der Formel II kann dadurch erfolgen, daß man
a) ein Insulinprodukt der Formel III oder IV 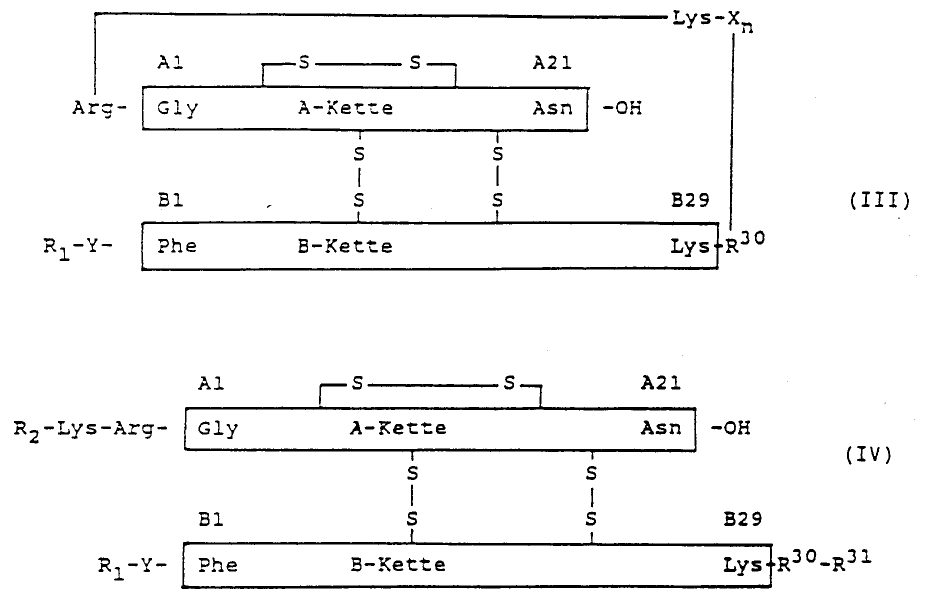 worin
   - X: = Reste gleicher oder verschiedener genetisch kodierbarer L-Aminosäuren,
   - Y: = Lys oder Arg,
   - n: = 0 oder ganze Zahl von 1 - 60,
   - R₁ und R₂: = H oder
   - gegebenenfalls derivatisierte - Reste einer natürlichen Aminosäure, oder
   - gegebenenfalls derivatisierte - Peptidreste aus 1 - 90, vorzugsweise 70 - 80 natürlichen Aminosäuren,
   - R³⁰ und R³¹: die gleiche Bedeutung wie in Formel II besitzen und die A- und B(1-29)-Ketten vorzugsweise die Sequenzen des Human-, Schweine- oder Rinderinsulins, insbesondere des Human- oder Schweineinsulins, aufweisen, 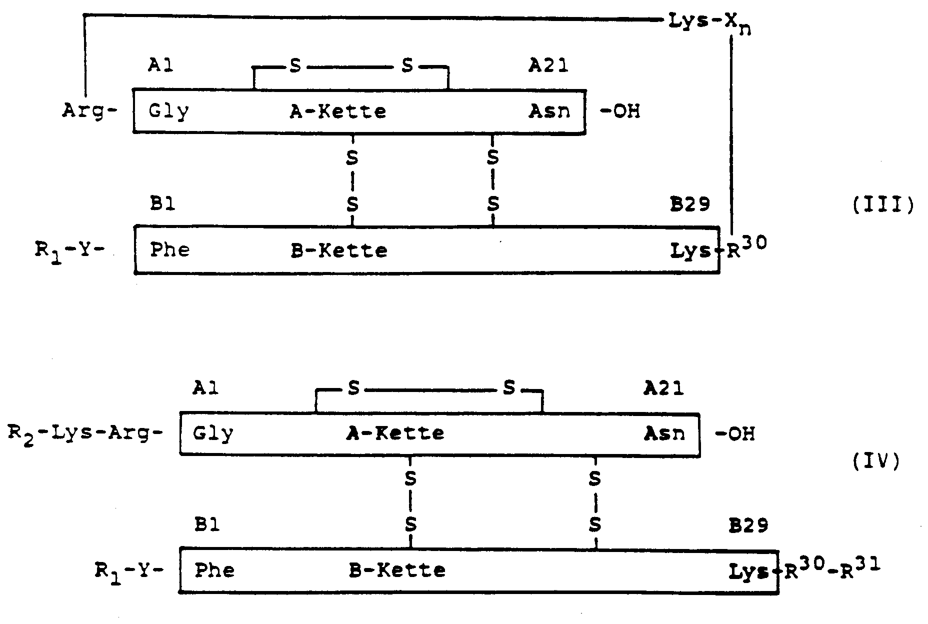
   worin
   - X: = Reste gleicher oder verschiedener genetisch kodierbarer L-Aminosäuren,
   - Y: = Lys oder Arg,
   - n: = 0 oder ganze Zahl von 1 - 60,
   - R₁ und R₂: = OH oder
   - gegebenenfalls derivatisierte - Reste einer natürlichen Aminosäure, oder
   - gegebenenfalls derivatisierte - Peptidreste aus 1 - 90, vorzugsweise 70 - 80 natürlichen Aminosäuren,
   - R³⁰ und R³¹: die gleiche Bedeutung wie in Formel II besitzen und die A- und B(1-29)-Ketten vorzugsweise die Sequenzen des Human-, Schweine- oder Rinderinsulins, insbesondere des Human- oder Schweineinsulins, aufweisen,
   mit Lysylendopeptidase in Kontakt bringt, wobei die Bindungen am C-terminalen Ende der Lys-Reste gespalten werden und
   gegebenenfalls - d.h. wenn Y = Arg - noch mit Trypsin oder einer trypsinähnlichen Protease versetzt, wobei der Teil R₁ - Y von der B-Kette abgespalten wird und ein A0-Arg-Des-B30-Insulinderivat der Formel IIa entsteht, und/oder daß man
b) zur Herstellung eines Insulinderivates der Formel IIb ein A0-Arg-Des-B30-Insulinderivat der Formel IIa in Gegenwart von Lysylendopeptidase oder Trypsin bzw. einer trypsinähnlichen Protease mit einer Verbindung der Formel V

   HR³⁰-R³¹ (V),

   worin R³⁰ und R³¹ die bei Formel IIb angegebenen Bedeutungen besitzen und worin vorhandene freie COOH-, OH-, SH-, NH₂-, Guanidino- und/oder Imidazol- Funktionen in an sich bekannter Weise geschützt vorliegen können, umsetzt, und
   anschließend gegebenenfalls vorhandene Schutzgruppen in an sich bekannter Weise abspaltet, oder daß man
c) ein A0-Arg-Des-Octapeptid (B23-30)-Insulin der Formel VI 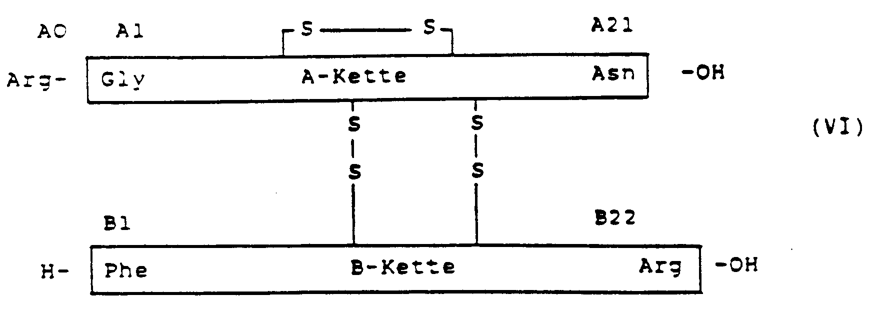 in Gegenwart von Trypsin oder einer trypsinähnlichen Protease umsetzt mit einer Verbindung der Formel VII
   H-Gly-Phe-Phe-Tyr-Thr-Pro-Lys-R³⁰-R³¹ (VII)
   in der R³⁰ und R³¹ die bei Formel II unter a) und b) angegebenen Bedeutungen besitzen und wobei vorhandene freie COOH-, OH-, SH-, NH₂-, Guanidino- und/oder Imidazol-Funktionen in an sich bekannter Weise geschützt vorliegen können und
   anschließend gegebenenfalls vorhandene Schutzgruppen in an sich bekannter Weise abspaltet, oder daß man
d) Insulin(derivate) der Formel II′ 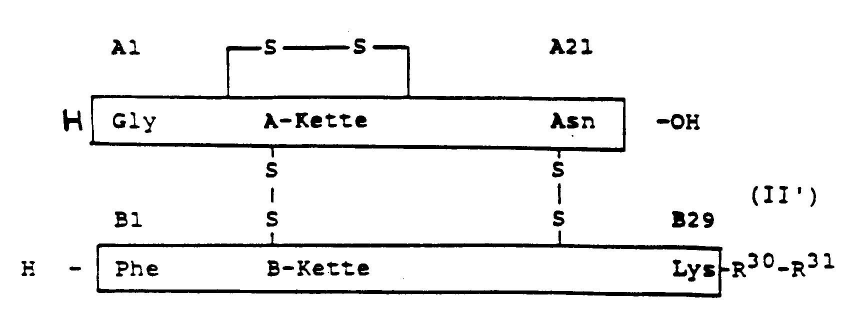 worin R³⁰ und R³¹ die gleiche Bedeutung wie in Formel II (a/b) besitzen
   und deren reaktive Aminogruppen - mit Ausnahme der Aminogruppe an A1-Gly - in bekannter Weise geschützt vorliegen,
   mit Arginin, dessen Aminogruppen ebenfalls in bekannter Weise geschützt sind und deren COOH-Gruppe gegebenenfalls in aktivierter Form vorliegt,
   umsetzt und anschließend die vorhandenen Schutzgruppen in bekannter Weise abspaltet.

Die erhaltenen Insulinderivate der Formel II können gewünschtenfalls in bekannter Weise in entsprechende physiologisch verträgliche Salze überführt werden.

Die vorerwähnten Verfahrensvarianten a) bis d) werden wie folgt näher erläutert.

### Variante a):

### Erläuterungen zu den Formeln der Ausgangsprodukte III und IV:

In Formel III kommt das Symbol X vor, welches gleiche oder verschiedene genetisch kodierbare Aminosäuren bedeutet. Genetisch kodierbar sind die folgenden Aminosäuren (jeweils in der L-Form):
Gly, Ala, Ser, Thr, Val, Leu, Ile, Asp, Asn, Glu, Gln, Cys, Met, Arg, Lys, His, Tyr, Phe, Trp, Pro.

Natürliche Aminosäuren - in den bzw. für die Reste R₁ und R₂ in Formel IV - sind u.a. Gly, Ala, Ser, Thr, Val, Leu, Ile, Asn, Gln, Cys, Met, Tyr, Phe, Pro, Hyp, Arg, Lys, Hyl, Orn, Cit und His.

Die Aminosäuren und Peptidketten können in üblicher Weise derivatisiert - d.i. mit in der Peptidchemie üblichen Schutzgruppen für die Amino- und/oder Carboxylgruppen versehen - sein.

Die Verbindungen der Formel III bzw. ihre Vorprodukte ohne Disulfidbrücken werden vorzugsweise gentechnisch, insbesondere nach dem aus der EP-A 0 289 936 bekannten Verfahren hergestellt. Die dort beschriebenen Vorprodukte sind Fusionsproteine aus Affenproinsulin mit (gegebenenfalls verkürztem) Tendamistat, verbunden über ein kurzes Peptidbrückenglied. Derartige Fusionsproteine können in Streptomycetenzellen exprimiert und in das Kulturmedium sekretiert werden, aus dem sie besonders leicht isoliert werden können. In der deutschen Patentanmeldung P 38 37 273.8 (HOE 88/F 313) werden besonders ausgestaltete Fusionsproteine vorgeschlagen, die dadurch gekennzeichnet sind, daß die A- und B-Kette des Insulins eine korrekte Disulfidbrückenverknüpfung aufweisen und das C-Peptid auf die Aminosäure Lysin verkürzt ist. Ganz analog dazu kann ein Fusionsprotein hergestellt werden, in dem die C-Kette auf die Aminosäure Arginin verkürzt ist. Eine besonders vorteilhaft Abwandlung des vorgeschlagenen Verfahrens besteht aber darin, in das Gen für das Fusionsprotein ein Codon für die Aminosäure Arginin an das vorhandene Codon für Lysin anzufügen, so daß also ein Fusionsprotein resultiert, dessen C-Kette aus Lys-Arg besteht.

Entsprechende Fusionsproteine können auch nach anderen, an sich bekannten Verfahren hergestellt werden, beispielsweise nach der EP-A-0 227 938, EP-A-0 229 998, EP-A-0 286 956 und der EP-A 0 290 005 in E. coli.

Die Verbindungen der Formel IV bzw. ihre Vorprodukte werden hergestellt, indem die Einzelketten 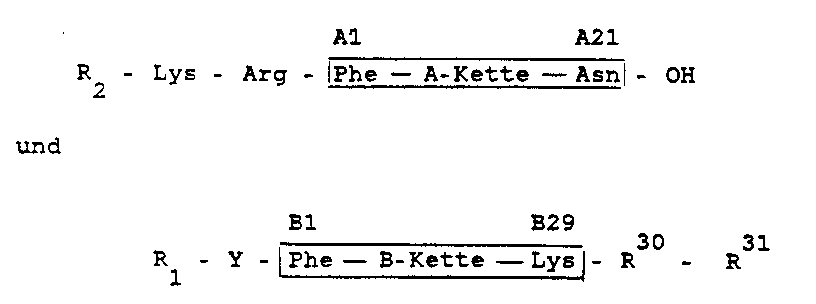
getrennt, vorzugsweise gentechnisch z.B. entsprechend den Verfahren gemäß EP-A- 0 289 936, EP-A-0 286 956, EP-A-0 229 998, EP-A-0 227 938 und EP-A-0 290 005 gebildet werden. Anschließend werden die Ketten, gegebenenfalls in geeigneter Form mit Schutzgruppen versehen, nach an sich bekannten Verfahren verknüpft. Solche Verfahren sind in der Literatur verschiedentlich beschrieben worden, z.B. von P.G. Katsoyannis et al. im J. Am. Chem. Soc. 85, 2863-2865 (1963).

Zur Durchführung der Verfahrensvariante a) wird dann die Verbindung der Formel III bzw. IV mit Lysylendopeptidase z.B. aus Lysobacter enzymogenes in wäßriger Lösung bzw. Suspension in Kontakt gebracht. Die eingesetzte (Gewichts-) Menge des reinen Enzyms beträgt vorzugsweise etwa 1/50 bis 1/10000, insbesondere zwischen etwa 1/100 bis 1/1000 der Menge des Ausgangs-Insulinproduktes der Formel III oder IV.

Der pH-Wert des Reaktionsansatzes kann in relativ weiten Grenzen schwanken; bevorzugt ist jedoch der Bereich zwischen etwa 5,5 und 10,5, insbesondere zwischen etwa 7 und 9.

Die Reaktionstemperatur ist vorzugsweise Raumtemperatur.

In Abhängigkeit hauptsächlich von der Enzymmenge beträgt die Reaktionszeit wenige Minuten bis mehrere Tage, vorzugsweise einige Stunden.

Das Enzym Lysylendopeptidase spaltet Peptidketten, welche die Aminosäure Lysin enthalten, an der Carboxylseite des Lysins. Aus den Verbindungen der Formeln III und IV entsteht daher - wenn Y = Lys - A0-Arg-Des-B30-Insulin, welches nach an sich bekannten Methoden gereinigt werden kann. Wenn in den Ausgangsverbindungen III bzw. IV Y = Arg, bleibt am N-terminalen Ende der B-Kette die Gruppierung R₁-Y-Phe erhalten. Die Abspaltung des Restes R₁-Y (= R₁-Arg) von der B-Kette muß dann durch eine Nachspaltung mit Trypsin oder einer trypsinähnlichen Protease erfolgen, was ohne oder auch nach der Isolierung des Zwischenproduktes R₁-Arg-Phe-...) geschehen kann. Die Isolierung und Reinigung des gebildeten AO-Arg-Des-B30-Insulinderivates wird dann wieder nach bekannten Methoden durchgeführt.

In der Literatur wird Lysylendopeptidase oft unter den trypsinähnlichen Endopeptidasen mit aufgeführt, oft auch daneben noch genannt. Im vorliegenden Fall wird die letztere Betrachtungsweise gewählt.

### Variante b):

Es handelt sich um eine Kupplungsreaktion, bei der die Verbindung der Formel V an das - etwa nach Verfahrensvariante a) erhaltene - A0-Arg-Des-B30-Insulinderivat (der Formel IIa) zu einem A0-Arg-Insulin bzw. -Insulinderivat der Formel IIb angeknüpft wird. Dies geschieht nach an sich bekannten Methoden analog wie z.B. für Transamidierungen beschrieben in der EP-B-00 56 951.

Wenn hier Ausgangsprodukte eingesetzt wurden, welche mit Schutzgruppen versehen waren, sind diese am Ende wieder auf bekannte Weise abzuspalten.

### Variante c):

Das Ausgangsprodukt der Formel VI kann analog zu den Ausgangsprodukten für Variante a) erhalten werden.

Die Verknüpfung mit dem Peptid der Formel VII geschieht auf an sich bekannte Weise, wie z.B. beschrieben von Inouye et al. in J. Am. Chem. Soc. 101, 751 - 752 (1979).

Auch hier sind - wenn mit Schutzgruppen versehene Ausgangsprodukte eingesetzt wurden - am Ende die Schutzgruppen wieder abzuspalten.

### Variante d):

Es handelt sich um die Anknüpfung von Arginin an ein Insulinderivat der Formel II′ in A0-Position.

Als Schutzgruppen für die Aminogruppen sowohl in dem Insulinderivat der Formel II′ - bei dem allerdings die Aminogruppe des A1-Gly ungeschützt bleiben muß - als auch in der Aminosäure Arginin kommen die in der Peptidchemie üblichen Aminogruppen - Schutzgruppen in Frage wie z.B. die Benzyloxycarbonyl-, die tert.-Butyloxycarbonyl- oder die Fluoren-9-yl methoxycarbonyl-Gruppe.

Wenn das Ausgangs-Arginin mit freier Carboxylgruppe eingesetzt wird, ist es zweckmäßig, die Verknüpfung mit dem A1-Gly mittels Carbodiimid durchzuführen. Ansonsten ist es zweckmäßig, die Carboxylgruppe zu "aktivieren", d.i. vor der eigentlichen Reaktion in eine aktivierte Form wie z.B. die Säurehalogenid oder -azid-Form zu überführen.

Die Insulinderivate der Formel II (a/b) und deren physiologisch verträgliche Salze dienen in erster Linie als Wirkstoffe für pharmazeutische Zubereitungen zur Behandlung des Diabetes mellitus.

Erfindungsgegenstand ist daher auch eine pharamzeutische Zubereitung, die durch einen Gehalt an mindestens einem Insulinderivat der Formel II und/oder mindestens einem von dessen physiologisch verträglichen Salzen in gelöster, amorpher und/oder kristalliner - vorzugsweise in amorpher und/oder kristalliner - Form gekennzeichnet ist.

Für diese pharmazeutische Zubereitung bevorzugte Insulinderivate der Formel II sind
A0-Arg-Des-B30-Humaninsulin,
A0-Arg-Humaninsulin,
A0-Arg-B31-Arg-OH-Humaninsulin und
A0-Arg-B31-Arg-B32-Arg-OH-Humaninsulin
und deren physiologisch verträglichen Salze.

Die pharmazeutische Zubereitung ist vorzugsweise eine Lösung oder Suspension zu Injektionszwecken mit einem pH-Wert zwischen etwa 3,0 und 9,0, vorzugsweise zwischen etwa 5,0 und 8,5, welche
ein geeignetes Isotoniemittel,
ein geeignetes Konservierungsmittel und gegebenenfalls einen geeigneten Puffer,
sowie gegebenenfalls auch eine bestimmte Zinkionen-Konzentration oder ein anderes Depotprinzip wie z.B. Protaminsulfat,
alles natürlich in steriler wäßriger Lösung oder Suspension, enthält. Die Gesamtheit der Zubereitungsbestandteile außer dem Wirkstoff bildet den Zubereitungsträger.

Geeignete Isotoniemittel sind z.B. Glycerin, Glukose, Mannit, NaCl, Calcium- oder Magnesium-Verbindungen wie z.B. CaCl₂, MgCl₂ etc.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 5,0 und 8,5, können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet.

Wenn die Zubereitung einen Zinkgehalt besitzt, ist ein solcher von etwa 1 µg bis 2 mg, insbesondere von etwa 5 µg bis 200 ug Zink/ml bevorzugt.

Zwecks Variation des Wirkungsprofils der erfindungsgemäßen Zubereitung können auch andere modifizierte (vgl. EP-B 132 769 und EP-B 132 770!) und/oder
unmodifizierte Insuline, vorzugsweise Rinder-, Schweine-oder Humaninsulin, insbesondere Humaninsulin, zugemischt werden.

Bevorzugte Wirkstoffkonzentrationen sind solche entsprechend etwa 1 bis 15000, weiter bevorzugt etwa 5 bis 1000 und insbesondere etwa 40 bis 400 Internationale Einheiten/ml.

Die pharmazeutische Zubereitung wird dadurch hergestellt, daß man mindestens ein Insulinderivat der Formel II und/oder mindestens eines von dessen physiologisch verträglichen Salzen, gegebenenfalls zusammen mit anderen modifizierten und/oder unmodifizierten Insulin(derivat)en, mit einem physiologisch unbedenklichen Träger sowie gegebenenfalls mit geeigneten Zusatz- und Hilfsstoffen in eine geeignete Darreichungsform bringt.

Die Erfindung wird nun durch die folgenden Beispiele näher erläutert.

### Herstellung von A0-Arg-Des-B30-Humaninsulin nach Verfahrensvarinate a)

### A) Herstellung eines Ausgangsproduktes der Formel III:

### A1)

Das in der Tabelle 1 wiedergegebene synthetische Gen (1) wird in an sich bekannter Weise nach der Phosphoamidit-Methode chemisch synthetisiert. Für die Codon-Auswahl wurde die Präferenz der Streptomyceten für G und C berücksichtigt. Wie bei dem für Affen-Proinsulin codierenden Gen der EP-A 0 289 936 (dort Tabelle 2) weist auch das Gen (1) gemäß Tabelle 1 am 5′-Ende eine für das Restriktionsenzym EcoRI typische überhängende Sequenz auf. Nach dem Strukturgen finden sich zwei Stopp-Codons und eine Linkersequenz mit der Erkennungsstelle für das Enzym SalI. Am 3′-Ende findet sich die überhängende Sequenz entsprechend dem Restriktionsenzym HindIII.

Das handelsübliche Plasmid pUC19 wird mit den Enzymen EcoRI und HindIII geschnitten und das synthetische Gen (1) gemäß Tabelle 1 hineinligiert. Man erhält so das Plasmid pI3 (2). Nach Amplifikation wird das synthetische Gen mit den Enzymen EcoRI und SalI als Fragment (3) herausgeschnitten und für die weiter unten beschriebene Konstruktion eingesetzt.

Das Plasmid pUC19 wird mit SmaI komplett verdaut und mit der in der Tabelle 2 wiedergegebenen Terminatorsequenz (4) ligiert. Plasmide, die diese Sequenz in der korrekten Orientierung enthalten, erhalten die Bezeichnung pT3 (5). Dieses Plasmid (5) wird mit EcoRI geöffnet und die Schnittstelle mit DNA-Polymerase (Klenow-Fragment) aufgefüllt. Durch Religieren erhält man das Plasmid pT4 (6). Dieses Plasmid wird mit den Enzymen SalI und SphI geöffnet und das große Fragment (7) isoliert.

Das Plasmid pKK400 (8) (vgl. EP-A 0 289 936, Figur 4, (20)) wird mit SphI und EcoRI geschnitten und das kleine Fragment (9) mit dem Tendamistatgen isoliert.

Durch Ligation der Fragmente (3), (7) und (9) erhält man das Plasmid pKK700 (10), in dem auf die Tendamistat-Sequenz das für 12 Aminosäuren codierende Brückenglied 
Phe Asn Ala Met Ala Thr Gly Asn Ser Asn Gly Lys
TTC AAT GCG ATG GCC ACC GGG ATT TCG AAC GGC AAG
AAG TTA CGC TAC CGG TGG CCC TAA AGC TTG CCG TTC
EcoRI
und hierauf das Gen für das modifizierte Proinsulin folgen. Die korrekte Anordnung wird durch Schneiden mit SphI und SstI kontrolliert, wobei aus dem ca. 3,5 kb großen Plasmid ein Fragment von 826 bp erhalten wird. Durch DNA-Sequenzierung nach der Di-desoxy-Methode wird die Richtigkeit der Sequenz bestätigt.

Genkonstruktionen, in denen das als C-Peptid fungierende Lys durch Arg ersetzt wird, werden analog hergestellt. Es wird hierzu das für Lys codierende Triplett durch CCC ersetzt. Man erhält analog das Plasmid pI5 und daraus den Vektor pKK800.

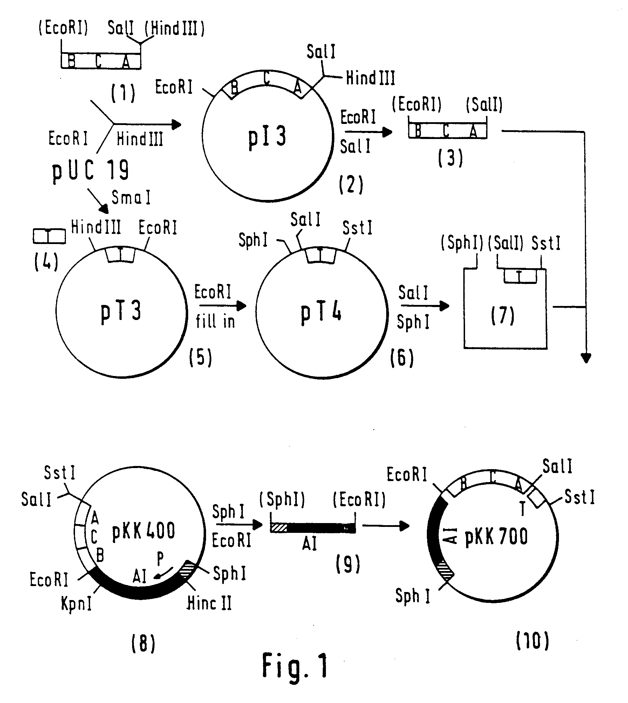

Figur 1 erläutert die Genkonstruktion gemäß der hier beschriebenen Methode A1; sie ist nicht maßstabsgerecht.

### A2)

Analog dem in der EP-A 0 289 936 beschriebenen Vektor PGF1 werden aus den Vektoren pKK700 und pKK800 die Expessions-Plasmide pGF4 und pGF5 hergestellt. Hierzu werden aus den Vektoren pKK700 und pKK800 durch Doppelverdauung mit SphI und SstI jeweils das Insert von 826 bzw. 823 bp isoliert und diese DNA-Fragmente in das mit den gleichen Enzymen gespaltene Expressions-Plasmid pIJ702 ligiert. Das Ligationsgemisch wird in S. lividans TK 24 transformiert und aus Thiostrepton-resistenten Transformanten, die Tendamistat-Aktivität zeigen (Plattentest), die Plasmid-DNA isoliert. Alle positiven Klone enthalten das eingesetzte Insert aus pKK700 bzw. pKK800.

Die Expression des codierten Fusionsproteins kann in bekannter Weise erfolgen. Inkubiert man den transformierten Stamm S. lividans TK 24 vier Tage bei 28°C im Schüttelkolben und trennt das Mycel von der Kulturlösung durch Zentrifugieren ab, so kann man das Fusionsprotein in der klaren Lösung wie folgt nachweisen:

10 bis 100 µl Lösung werden mit 20 bis 200 µl 15 %iger Trichloressigsäure versetzt, das ausgefallene Protein wird durch Zentrifugieren angereichert, gewaschen und in SDS-haltigem Probenpuffer (U. Laemmli, Nature 227 (1970) 680-685) aufgenommen. Nach 2 Minuten Inkubation bei 90°C trennt man elektrophoretisch auf einem 10-17 %igen SDS-Polyacrylamidgel auf. Man erhält ein Protein vom Molekulargewicht 15 kD, also im erwarteten Molekulargewichtsbereich für das Fusionsprotein aus Tendamistat und Proinsulin. Das Fusionsprotein - ein unter Formel III fallendes Produkt - reagiert sowohl mit Antikörpern gegen Tendamistat als auch mit Antikörpern gegen Insulin.

### A3)

### Stammhaltung und Fermentation

Stämme von S. lividans, die das rekombinante Plasmid pGF4 gemäß A2) enthalten, werden auf Nähragarplatten ausgestrichen, die als komplexes Nährmedium R2YE Medium (Hopwood et al., Genetic Manipulation of Streptomyces: A Laboratory Manual; John Innes Foundation, Norwich, England; 1985) enthalten und bei Temperaturen von 25 bis 30°C, vorzugsweise bei 28°C, bebrütet werden. Zur Stabilisierung der Plasmide enthält das Sporulationsmedium als Selektionszusatz Thiostrepton in einer Konzentration von 20 µg/ml. Nach erfolgter Sporulation werden die Sporen durch Überschichten der Platten mit Wasser und Ultraschallbehandlung abgeerntet. Nach Titrierung der Sporen wird eine Lösung von 10¹⁰ Sporen/ml in 20 % wäßrigem Glycerin hergestellt und bei -20°C aufbewahrt.

### A4)

### Anlegen von Kulturen

Als Vorkulturmedium wird ein komplexes Nährmedium bestehend aus Sojamehl (20 g/l), Glucose (10 g/l), Maisstärke (2 g/l), Harnstoff (1 g/l), Ammoniumnitrat (1 g/l), Malzextrakt (5 g/l) und KH₂PO₄ (2 g/l) und als Selektionszusatz Thiostrepton in einer Konzentration von 10 bis 50 µg/l verwendet. Als Inoculum werden 5 x 10⁹ Sporen/l Endvolumen eingesetzt. Die Vorkultur wird mit 220 rpm bei 27°C geschüttelt und nach 60 Stunden in einer Verdünnung von 1:20 in die Hauptkultur überimpft.

Als Hauptkultur wird ein komplexes Nährmedium verwendet, dessen pH-Wert auf 7,2 eingestellt ist und das aus löslicher Stärke (40 g/l), Cornsteep flüssig (4 g/l), Magermilchpulver (7 g/l), Glucose (10 g/l), (NH₄)₂SO₄ (12 g/l) und Sojamehl (4 g/l) besteht.

Die Fermentation erfolgt 48 Stunden in üblichen Rührkesselfermentern bei einer Begasung von 0,5 vvm Luft, einer Rührung von 200 - 240 rpm und 25°C. Nach Beendigung der Fermentation wir die Kulturlösung weiterbehandelt, indem die Zellpellets vom Kulturfiltrat durch Abnutschen über eine geschlossene Nutsche zur Vermeidung von Aerosolbildung abgetrennt werden. Das klare Kulturfiltrat enthält das gewünschte Fusionsprotein.

Die Ausbeute an Fusionsprotein ist um bis zu 20 % höher, wenn der pH- Wert der Hauptkulturlösung mit NaOH und 3 % Phosphorsäure bei 6,5 bis 7,2, vorzugsweise 6,9, konstant gehalten wird.

### A5)

### Isolierung des Fusionsproteins PTF1

30 l Kulturlösung der Streptomyces lividans-Fermentation zur Bildung des Plasmid-kodierten Fusionsproteins, gewonnen entsprechend dem Verfahren gemäß EP-A-0 289 936, im folgenden PTF1, werden zur Abtötung der Kultur mit 4,0 g p-Chlor-metakresol versetzt und 30 Minuten stehen gelassen. Nach dieser Zeit wird auf einer Filterpresse die Zellmasse abgetrennt und das Filtrat unter Kühlung mit Trichloressigsäure auf pH 4,0 gestellt. Nach 3 Stunden wird der Niederschlag durch Zentrifugation gesammelt. Anschließendes Ausrühren mit der 10fachen Menge Aceton führt zur Entfettung Die Acetonphase wird abfiltriert und verworfen. Durch Aufschlemmen in 6-molarer Harnstofflösung und Einstellen des pH-Wertes auf 7,5 wird das Protein PTF1 in Lösung gebracht und vom ungelöst gebliebenem Material durch erneutes zentrifugieren separiert. Die klare flüssige Phase kann direkt auf eine Q-^{(R)} Sepharose-Säule aufgetragen werden, die mit Tris/HCl-Puffer (pH 7,5) in 3-molarem Harnstoff äquilibriert wurde. Die Säule (5 cm Durchmesser, 15 cm hoch) enthält 300 ml Ionenaustauscher. Das Eluieren geschieht mit einer 0 bis 0,5-molaren NaCl-Harnstofflösung.

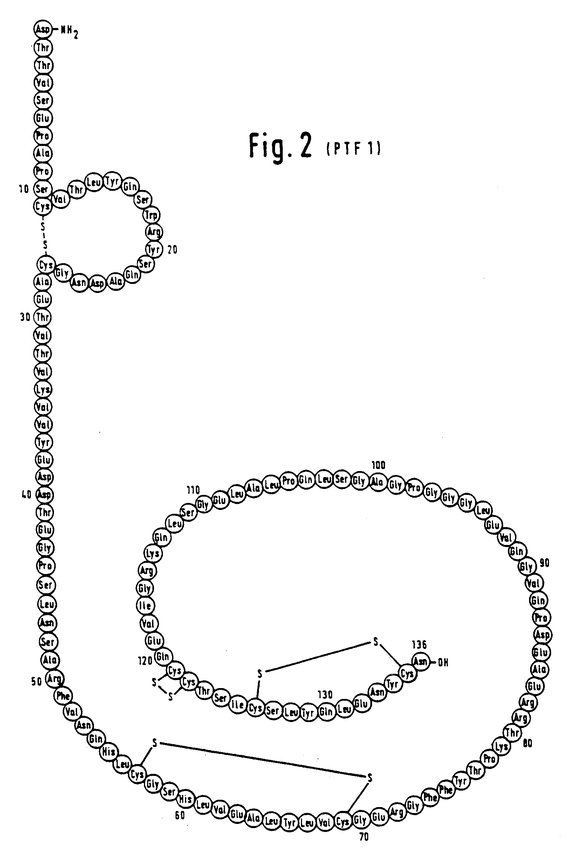

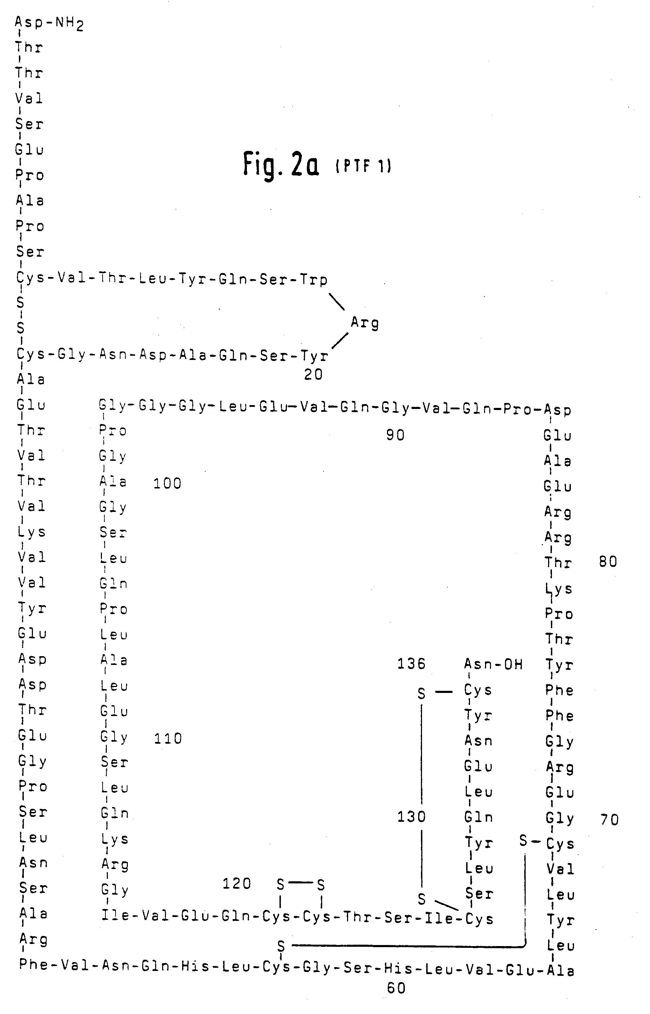

Die PTF1-haltigen Fraktionen werden gesammelt und durch Dialyse entsalzt. 300 mg des angereicherten Produktes trägt man dann auf eine Macrobore ^{(R)} Nucleosil 120 - 10 C4 HPLC-Säule auf und eluiert mit 0,1 %iger Trifluoressigsäure, der teigenden Mengen Acetonitril zugefügt werden. Mit 36 %igem Acetonitril wird das Protein PTF1 von der Säule gelöst. Die entsprechenden Fraktionen werden im Vakuum vom Lösemittel befreit. Es resultieren 186 mg reines PTF1. Die Strukturformel ist in Figur 2 wiedergegeben.

### A6)

### Umfaltung des Fusionsproteins PTF1

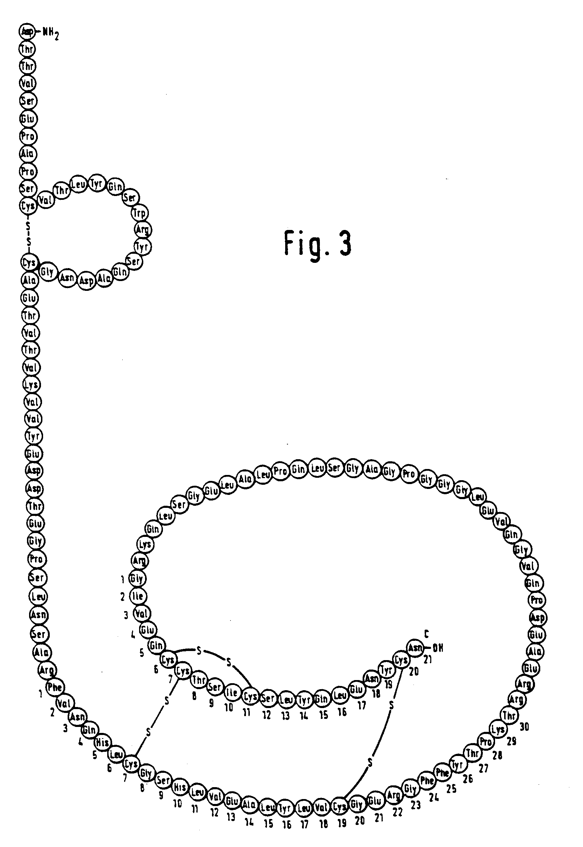

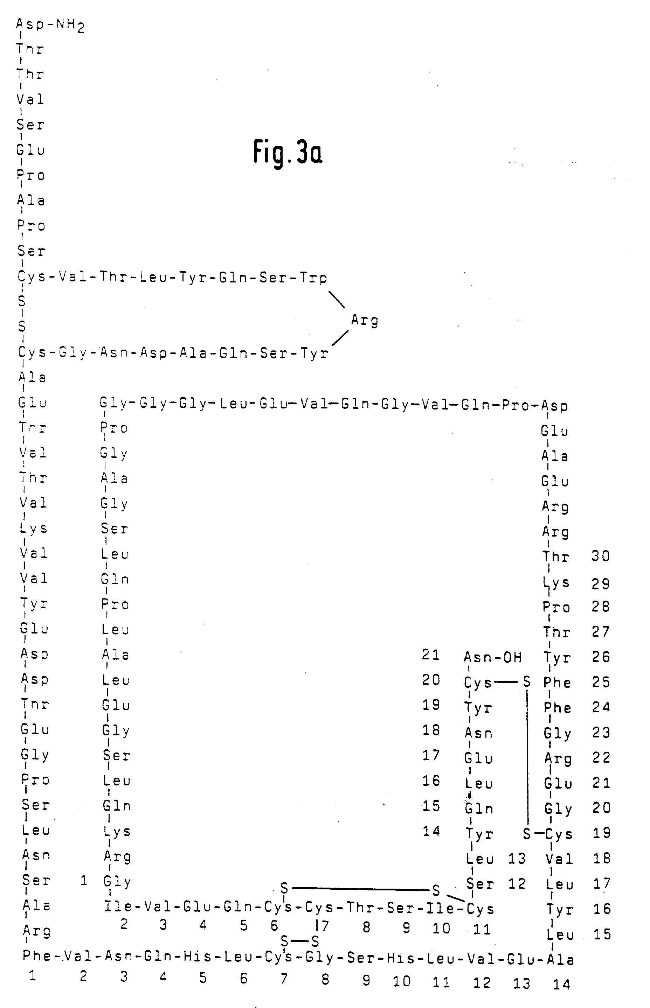

34 mg PTF1 werden in 70 ml 8-molarem Harnstoff (pH 8,6) gelöst und 5 Minuten mit Helium begast. Danach werden 760 µl β-Mercaptoethanol (pH 10,5) zugesetzt. Nach 30 Minuten Reduktionszeit wird während 16 Stunden unter Sauerstoffausschluß gegen Glycin/NaOH-Puffer (pH 10,5) dialysiert. Nach dieser Zeit wird mit Luft begast, anschließend sauer gestellt und über Nucleosil 120 10 Ca getrennt. Die Struktur des umgefalteten Produktes ist in Figur 3 wiedergegeben.
Die Ausbeute beträgt 8,2 mg, entsprechend 24 % der Theorie.

### A7)

### Isolierung des Proteins PGF4

Das nach A4) gebildete Protein wird entsprechend A5) aufgearbeitet.

### Erfindungsbeispiel 1:

### Enzymatische Spaltung des umgefalteten Proteins PTF1

Die 8,2 mg umgefaltetes PTF1 werden in 1 ml Trispuffer (pH 8,0) gelöst und 10 µl einer 1 mg/ml starken Lysylendopeptidase (LEP) aus Lysobacter enzymogenes hinzugegeben. Die Lösung läßt man 2 Stunden bei Raumtemperatur stehen und trennt anschließnd erneut auf der Nucleosil 120 - 5 Ca-HPLC-Säule auf.
Ausbeute: 3,8 mg, entsprechend 92 % der Theorie.

Die 3,8 mg Insulinvorprodukt werden in 1 ml Trispuffer (pH 8,4) gelöst und 5 µl einer 1 mg/ml enthaltenden Trypsinlösung hinzugefügt. Nach 90 Minuten wird die Reaktion durch Senken des pH-Wertes auf 3,0 gestoppt und auf einer Nucleosil 120 - 5 C4-HPLC-Säule mit dem System 0,05 % Trifluoressigsäure in Wasser/Acetonitril getrennt. Die Gefriertrocknung der aktiven Fraktionen ergibt 2,1 mg reines A0-Arg-Des-Thr-B30-Humaninsulin. Der isoelektrische Punkt des neuen Materials beträgt ca. 6,2.

### Erfindungsbeispiel 2:

### Gewinnung des A0-Arg-Des-Thr-B30-Insulin aus E. coli-Proteinen

### Ausgangsprodukt:

Durch Fermentation eines E.coli-Stammes, der mit einem Plasmid transformiert wurde, das dem Plasmid pWZIP dMdC (EP-A 0 286 956, Beispiel 3) entspricht, aber für ein Proinsulin mit einer auf Arg verkürzten C-Kette codiert, und die nachfolgende Isolierung sowie die Umwandlung des gebildeten Proteins führen zu einem analogen Insulinvorprodukt.

### Erfindung:

208 mg dieses Materials werden in 100 ml Tris/HCl-Puffer, 0,1 m (pH 8,4), gelöst und mit 1 mg einer im gleichen Puffer gelösten Lysylendopeptidase aus Lysobacter enzymogenes versetzt. Man läßt 4 Stunden bei Raumtemperatur unter gelengentlichem Umrühren reagieren. Die Reaktion wird regelmäßig durch HPLC überprüft. Nach der angegebenen Zeit ist das C-Peptid zu über 95 abgespalten. Daraufhin werden zum Reaktionsansatz 416 µl Trypsinlösung (Konzentration 1 mg/ml) gegeben und weitere 3 Stunden reagieren lassen. Die Reaktion wird anschließend durch Sauerstellen mit Trifluoressigsäure gestoppt und der Ansatz auf einer Nucleosil RP-C4-Säule (25 cm x 4,8 cm) im Lösungsmittelsystem Wasser-Trifluoressigsäure (0,1 %) - Acetonitril aufgetrennt. Es resultieren nach der Gefriertrocknung der Insulin- haltigen Fraktion 88 mg A0-Arg-Des-Thr-B30-Humaninsulin.

### Herstellung von A0-Arg-Humaninsulin nach Verfahrensvariante b):

### Erfindungsbeispiel 3:

### Umsetzung des A0-Arg-Des-Thr-B30-Insulins zu AO-Arg-Insulin

13 mg A0-Arg-B30-Des-Thr-Insulin, gewonnen nach Erfindungsbeispiel 1 oder 2 werden mit 0,25 ml 10 M Essigsäure und 0,65 ml 1,54 M L-Threoninmethylester in DMSO/Butandiol - 1,3, (1:1) gelöst. Dazu gibt man 150 µl Lysylendopeptidase aus Lysobacter enzymogenes (Calbiochem No. 440275), vorgelöst in Wasser (14 mg/ml). Der resultierende pH beträgt ca. 5,3. Der Ansatz bleibt bei Raumtemperatur 2 Stunden stehen. Es entstehen zu 94 % der entsprechende Methylester des A0-Arg-Humaninsulins. Der Umsatz wird durch HPLC verfolgt. Das Protein wird durch Zusatz von 1 ml Methanol und 4 ml Methyl-tert.-butylether ausgefällt. Der Niederschlag einmal mit Ether gewaschen und getrocknet. Zur Abspaltung des Methylesters wird das Produkt in 10 ml Glycinpuffer, 0,1 M + 10 mM Butylamin bzw. pH = 10,0 einige Stunden stehengelassen.

Danach wird wieder ausgefällt und der Niederschlag in 0,1 % Trifluoressigsäure aufgenommen und über präparative HPLC gereinigt. Die reinen Fraktionen enthalten A0-Arg-Humaninsulin.

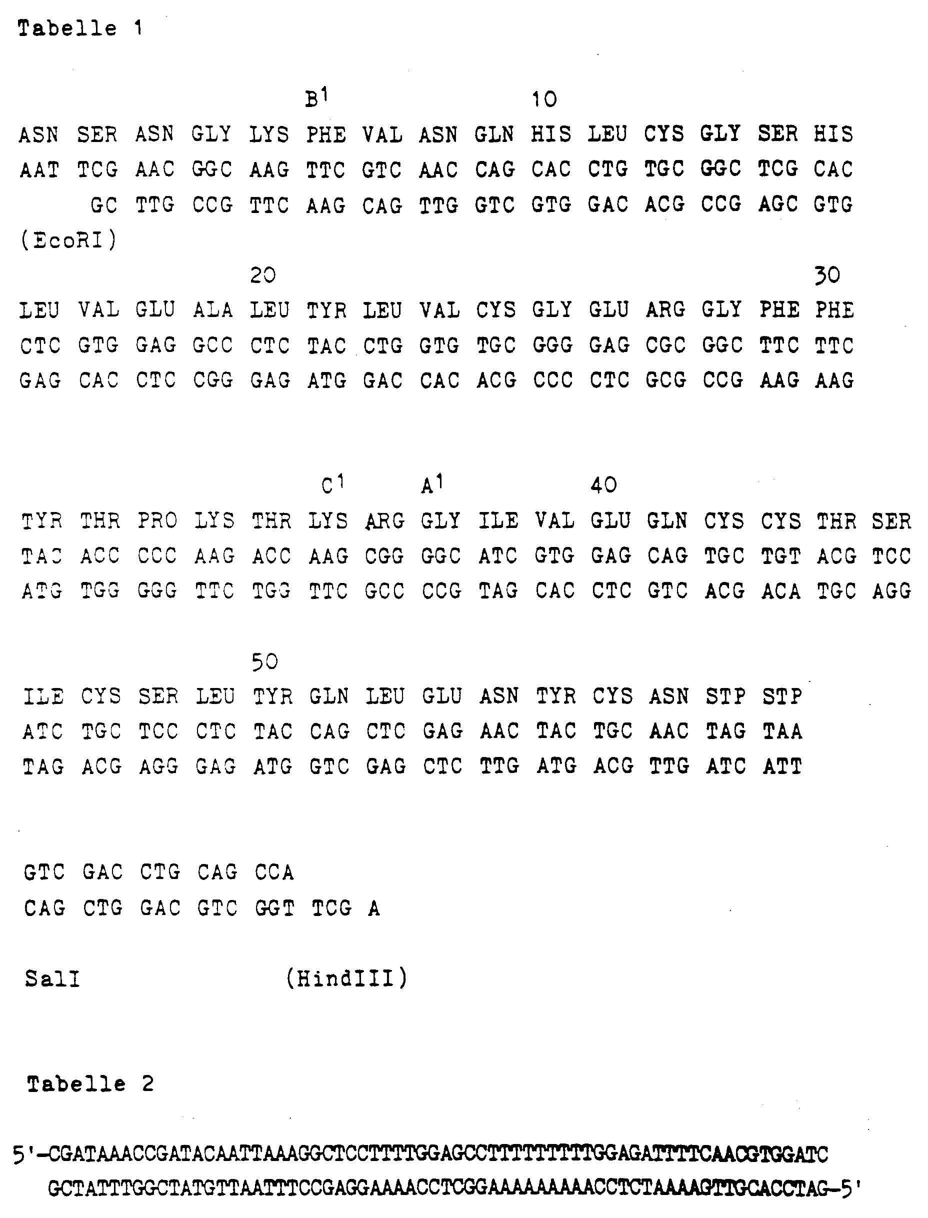

## Patentansprüche (Patentansprüche für folgende(n) Vertragsstaat(en): DE, GB, FR, IT, NL, SE, LI, CH, BE, AT, LU)

1. Ein im wesentlichen gereinigtes Insulinderivat der Formel II 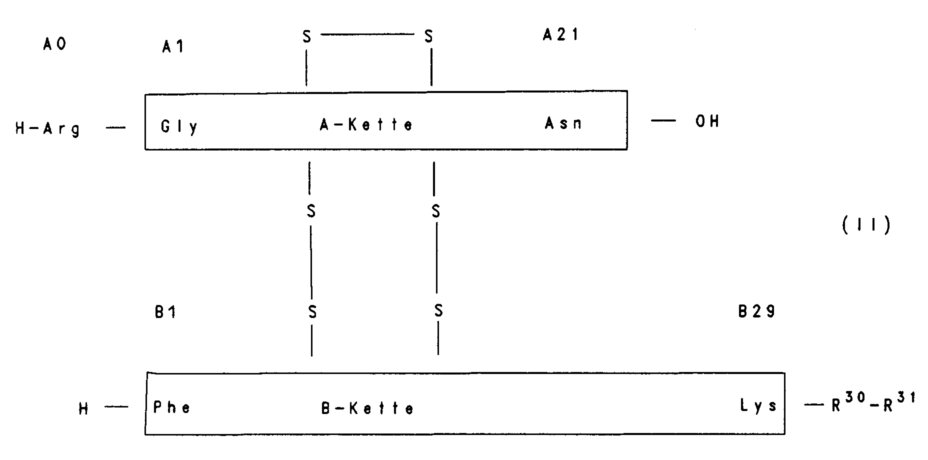 in welcher
a)
R³⁰ und R³¹ zusammen OH bedeuten oder
b)
R³⁰ bedeutet Rest einer neutralen, genetisch kodierbaren L-Aminosäure und
R³¹ bedeutet OH oder 1 bis 3 L-Aminosäuren,
sowie physiologisch verträgliche Salze des Insulinderivats der Formel II, mit Ausnahme der Fälle, in welchen gleichzeitig R³⁰ Ala und R³¹ OH ist sowie die A- und B-Ketten die Sequenzen des Rinderinsulins sind.

2. Insulinderivate und deren physiologisch verträgliche Salze nach Anspruch 1,
dadurch gekennzeichnet, daß in Formel II
R³⁰ + R³¹ = OH.

3. Insulinderivate und deren physiologisch verträgliche Salze nach Anspruch 1,
dadurch gekennzeichnet, daß in Formel II
für den Fall b) R³⁰ = Rest von Ala, Thr oder Ser, insbesondere von Thr, und
R³¹ = OH, Arg-OH oder Arg-Arg-OH.

4. Insulinderivate und deren physiologisch verträgliche Salze nach einem oder mehreren der Ansprüche 1 bis 3, dadurch gekennzeichnet, daß in Formel II die A-Kette und die B-Kette (B1 - B29) die Sequenzen des Human-, Schweine- oder Rinder-Insulins, vorzugsweise des Human- oder Schweine-Insulins sind.

5. Insulinderivate nach einem oder mehreren der Ansprüche 1 bis 4, gekennzeichnet durch einen isoelektrischen Punkt zwischen 5,5 und 9,0.

6. Verfahren zur Herstellung von Insulinderivaten der Formel II gemäß der Definition in einem oder mehreren der Ansprüche 1 bis 5,
dadurch gekennzeichnet, daß man
ein Insulinprodukt der Formel III oder IV 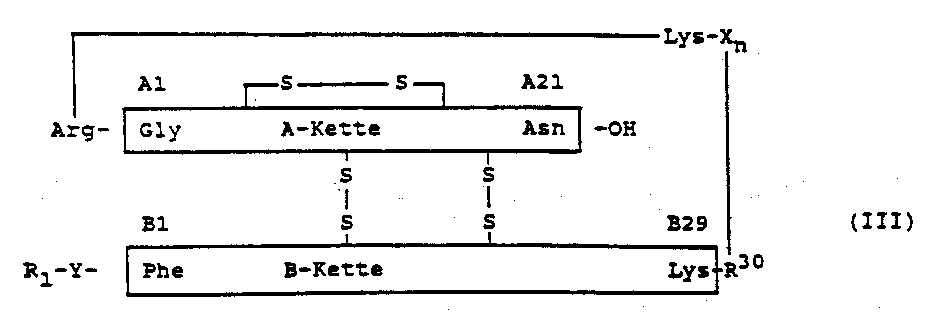 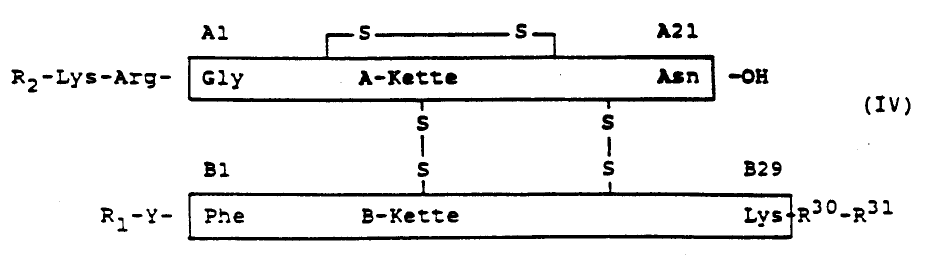 worin
X = Reste gleicher oder verschiedener genetisch kodierbarer L-Aminosäuren,
Y = Lys oder Arg,
n = 0 oder ganze Zahl von 1 - 60,
R₁ und R₂ = OH oder
- gegebenenfalls derivatisierte - Reste einer natürlichen Aminosäure, oder
- gegebenenfalls derivatisierte - Peptidreste aus 1 - 90, vorzugsweise 70 - 80 natürlichen Aminosäuren,
R³⁰ und R³¹ die gleiche Bedeutung wie in Formel II besitzen und die A- und B(1-29)-Ketten vorzugsweise die Sequenzen des Human-, Schweine- oder Rinderinsulins, insbesondere des Human- oder Schweineinsulins, aufweisen, mit Lysylendopeptidase in Kontakt bringt, wobei die Bindungen am C-terminalen Ende der Lysylreste gespalten werden,
und gegebenenfalls - d.h. wenn Y = Arg - noch mit Trypsin oder einer trypsinähnlichen Endopeptidase versetzt, wobei der Teil R₁-Y von der B-Kette abgespalten wird und ein A0-Arg-Des-B30-Insulinderivat der Formel IIa entsteht, und/oder daß man
b) zur Herstellung eines Insulinderivats der Formel IIb ein A0-Arg-Des-B30-Insulinderivat der Formel IIa in Gegenwart von Lysylendopeptidase oder Trypsin bzw. einer trypsinähnlichen Endopeptidase umsetzt mit einer Verbindung der Formel V
H-R³⁰-R³¹ (V),
worin R³⁰ und R³¹ die bei Formel IIb angegebenen Bedeutungen besitzen
und worin vorhandene freie COOH-, OH-, SH-, NH₂-, Guanidino- und/oder Imidazol-Funktionen in an sich bekannter Weise geschützt vorliegen können, und anschließend gegebenenfalls vorhandene Schutzgruppen in an sich bekannter Weise abgespalten werden, oder daß man
c) ein A0-Arg-Des-Octapeptid (B23-30)-Insulin der Formel VI 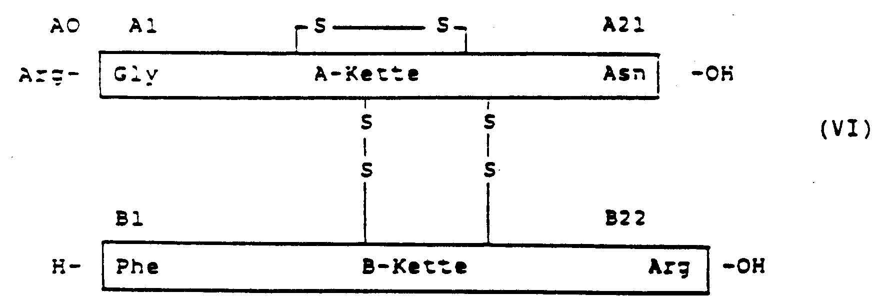 in Gegenwart von Trypsin oder einer trypsinähnlichen Endopeptidase mit einer Verbindung der Formel VII H-Gly-Phe-Phe-Tyr-Thr-Pro-Lys-R³⁰-R³¹ (VII) worin R³⁰ und R³¹ die gleiche Bedeutung wie in Formel II (a/b) besitzen
und worin vorhandene freie COOH-, OH-, SH-, NH₂-, gunaidino- und/oder Imidazol-Funktionen in an sich bekannter Weise geschützt vorliegen können, umsetzt und anschließend gegebenenfalls vorhandene Schutzgruppen in an sich bekannter Weise abspaltet, oder daß man
d) Insulin(derivat)e der Formel II′ 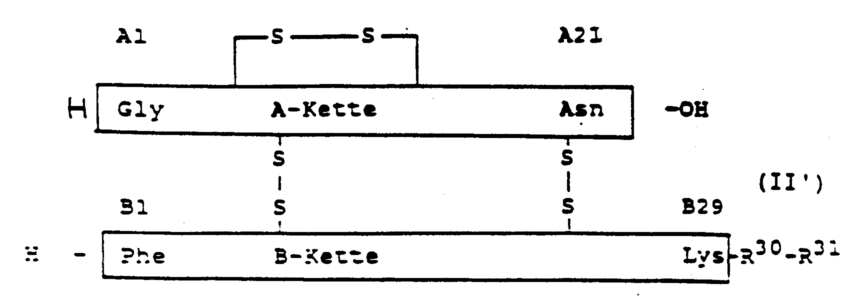 worin R³⁰ und R³¹ die gleiche Bedeutung wie in Formel II (a/b) besitzen
und worin reaktive Aminogruppen - mit Ausnahme der Aminogruppe an A1-Gly - in bekannter Weise geschützt vorliegen,
mit Arginin, dessen Aminogruppen ebenfalls in bekannter Weise geschützt sind und dessen Carboxylgruppe gegebenenfalls in aktivierter Form vorliegt,
umsetzt und anschließend die vorhandenen Schutzgruppen in bekannter Weise abspaltet.

7. Verwendung der Insulinderivate und deren physiologisch verträglicher Salze gemäß einem oder mehreren der Ansprüche 1 bis 5 als Wirkstoffe für pharmazeutische Zubereitungen zur Behandlung von Diabetes mellitus.

8. Pharmazeutische Zubereitung, gekennzeichnet durch einen Gehalt an mindestens einem Insulinderivat der Formel II und/oder mindestens eines von deren physiologisch verträglichen Salzen gemäß der Definition in einem oder mehreren der Ansprüche 1 bis 5 in gelöster, amorpher und/oder kristalliner - vorzugsweiser in amorpher und/oder kristalliner - Form.

9. Pharmazeutische Zubereitung nach Anspruch 8,
gekennzeichnet durch einen Gehalt an mindestens einer der folgenden - unter die Formel II fallenden - lnsulinderivate:
A0-Arg-Des-B30-Humaninsulin,
A0-Arg-Humaninsulin,
A0-Arg-B31-Arg-OH-Humaninsulin und
A0-Arg- B31-Arg- B32-Arg-OH-Humaninsulin
sowie deren physiologisch verträglichen Salze.

10. Phrmazeutische Zubereitung nach Anspruch 8 oder 9 als Injektionslösung bzw. -suspension mit einem pH-Wert zwischen etwa 3,0 und 9,0, vorzugsweise etwa 5,0 und 8,5.

11. Verfahren zur Herstellung einer pharmazeutischen Zubereitung gemäß einem oder mehreren der Ansprüche 8 bis 10, dadurch gekennzeichnet, daß man mindestens ein Insulinderivat der Formel II und/oder mindestens eines von deren physiologisch verträglichen Salzen, gegebenenfalls mit anderen modifizierten und/oder unmodifizierten Insulin(derivat)en, mit einem physiologisch annehmbaren Träger sowie gegebenenfalls geeigneten Zusatz- und/oder Hilfsstoffen in eine geeignete Darreichungsform bringt.

## Patentansprüche (Patentansprüche für folgende(n) Vertragsstaat(en): ES, GR)

1. Verfahren zur Herstellung eines im wesentlichen gereinigten Insulinderivats der Formel II 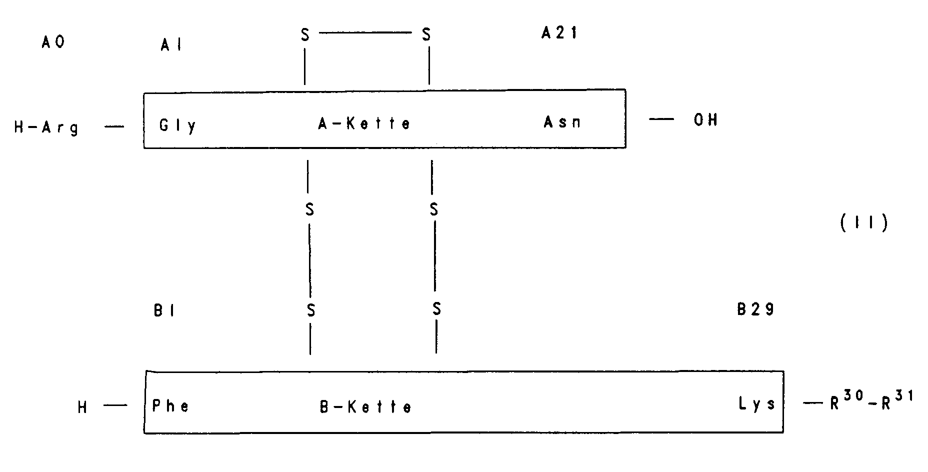 in welcher
a)
R³⁰ und R³¹ zusammen OH bedeuten oder
b)
R³⁰ bedeutet Rest einer neutralen, genetisch kodierbaren L-Aminosäure und
R³¹ bedeutet OH oder 1 bis 3 L-Aminosäuren,
sowie physiologisch verträgliche Salze des Insulinderivats der Formel II, mit Ausnahme der Fälle, in welchen gleichzeitig R³⁰ Ala und R³¹ OH ist sowie die A- und B-Ketten die Sequenzen des Rinderinsulins sind, dadurch gekennzeichnet, daß man
a) ein Insulinprodukt der Formel III oder IV 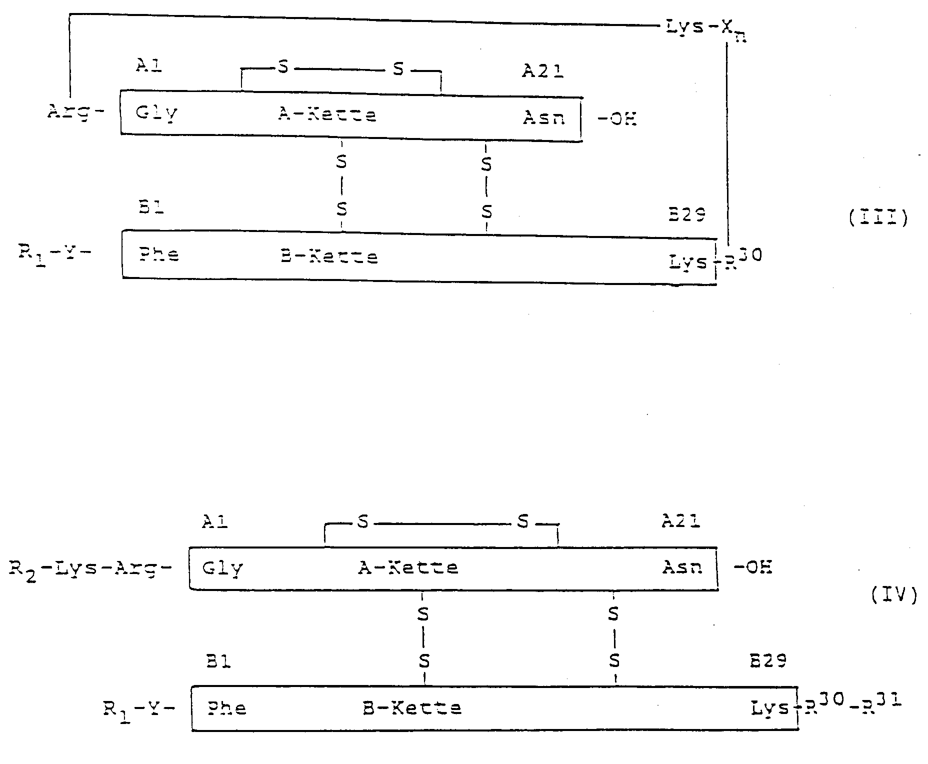 worin
X = Reste gleicher oder verschiedener genetisch kodierbarer L-Aminosäuren,
Y = Lys oder Arg,
n = 0 oder ganze Zahl von 1 - 60,
R₁ und R₂ = H oder
- gegebenenfalls derivatisierte - Reste einer natürlichen Aminosäure, oder
- gegebenenfalls derivatisierte - Peptidreste aus 1 - 90, vorzugsweise 70 - 80 natürlichen Aminosäuren,
R³⁰ und R³¹ die gleiche Bedeutung wie in Formel II besitzen und die A- und B(1-29)-Ketten vorzugsweise die Sequenzen des Human-, Schweine- oder Rinderinsulins, insbesondere des Human- oder Schweineinsulins, aufweisen, mit Lysylendopeptidase in Kontakt bringt, wobei die Bindungen am C-terminalen Ende der Lysylreste gespalten werden,
und gegebenenfalls - d.h. wenn Y = Arg - noch mit Trypsin oder einer trypsinähnlichen Endopeptidase versetzt, wobei der Teil R₁-Y von der B-Kette abgespalten wird und ein A0-Arg-Des-B30-Insulinderivat der Formel IIa entsteht, und/oder daß man
b) zur Herstellung eines Insulinderivats der Formel IIb ein A0-Arg-Des-B30-Insulinderivat der Formel IIa in Gegenwart von Lysylendopeptidase oder Trypsin bzw. einer trypsinähnlichen Endopeptidase umsetzt mit einer Verbindung der Formel V
H-R³⁰-R³¹ (V),
worin R³⁰ und R³¹ die bei Formel IIb angegebenen Bedeutungen besitzen
und worin vorhandene freie COOH-, OH-, SH-, NH₂-, Guanidino- und/oder Imidazol-Funktionen in an sich bekannter Weise geschützt vorliegen können, und anschließend gegebenenfalls vorhandene Schutzgruppen in an sich bekannter Weise abgespalten werden, oder daß man
c) ein A0-Arg-Des-Octapeptid (B23-30)-Insulin der Formel VI 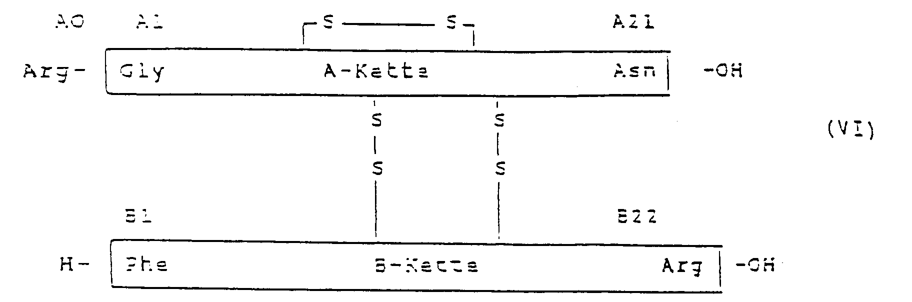 in Gegenwart von Trypsin oder einer trypsinähnlichen Endopeptidase mit einer Verbindung der Formel VII
H-Gly-Phe-Phe-Tyr-Thr-Pro-Lys-R³⁰-R³¹ (VII)
worin R³⁰ und R³¹ die gleiche Bedeutung wie in Formel II (a/b) besitzen
und worin vorhandene freie COOH-, OH-, SH-, NH₂-, Guanidino- und/oder Imidazol-Funktionen in an sich bekannter Weise geschützt vorliegen können, umsetzt und anschließend gegebenenfalls vorhandene Schutzgruppen in an sich bekannter Weise abspaltet, oder daß man
d) Insulin(derivat)e der Formel II' 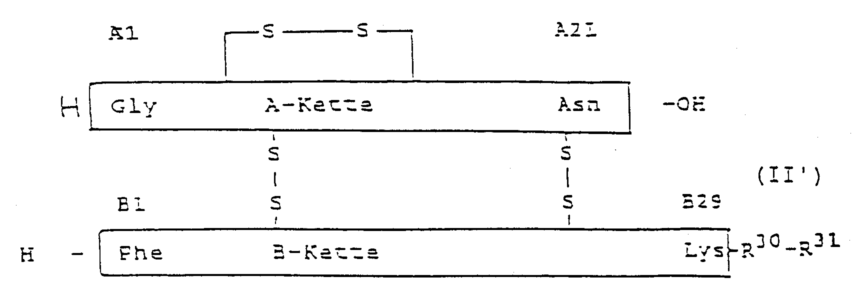 worin R³⁰ und R³¹ die gleiche Bedeutung wie in Formel II (a/b) besitzen und worin reaktive Aminogruppen - mit Ausnahme der Aminogruppe an Al-Gly - in bekannter Weise geschützt vorliegen,
mit Arginin, dessen Aminogruppen ebenfalls in bekannter Weise geschützt sind und dessen Carboxylgruppe gegebenenfalls in aktivierter Form vorliegt, umsetzt und anschließend die vorhandenen Schutzgruppen in bekannter Weise abspaltet,
und daß man gegebenenfalls die Endprodukte aller Verfahrensvarianten a-d in deren physiologisch verträgliche Salze überführt.

2. Verfahren nach Anspruch 1, dadurch gekennzeichnet, daß man Insulinderivate der Formel IIa,
worin R³⁰ + R³¹ zusammen = OH,
herstellt und gegebenenfalls in deren physiologisch verträgliche Salze überführt.

3. Verfahren nach Anspruch 1, dadurch gekennzeichnet, daß man Insulinderivate der Formel IIb worin R³⁰ = Rest von Ala, Thr oder Ser, insbesondere von Thr, und R³¹ = OH, Arg-OH oder Arg-Arg-OH, herstellt und gegebenenfalls in deren physiologisch verträgliche Salze überführt.

4. Verfahren nach einem oder mehreren der Ansprüche 1 bis 3, dadurch gekennzeichnet, daß man Verbindungen der Formel II, worin die A-Kette und die B-Kette (B1 - B29) die Sequenzen des Human-, Schweine- oder Rinder-Insulins, vorzugsweise des Human- oder Schweine-Insulins sind, herstellt und gegebenenfalls in deren physiologisch verträgliche Salze überführt.

5. Verfahren nach einem oder mehreren der Ansprüche 1 bis 4, dadurch gekennzeichnet, daß man Verbindungen der Formel II mit einem isoelektrischen Punkt zwischen 5,5 und 9,0 herstellt.

6. Verwendung der Insulinderivate der Formel II(a/b) gemäß der Definition in Anspruch 1 und deren physiologisch verträglicher Salze als Wirkstoffe für pharmazeutische Zubereitungen zur Behandlung von Diabetes mellitus.

7. Verfahren zur Herstellung einer pharmazeutischen Zubereitung, dadurch gekennzeichnet, daß man mindestens ein Insulinderivat der Formel II (a/b) gemäß der Definition in Anspruch 1 und/oder mindestens eines von deren physiologisch verträglichen Salzen, gegebenenfalls mit anderen modifizierten und/oder unmodifizierten Insulin(derivat)en, mit einem physiologisch annehmbaren Träger sowie gegebenenfalls geeigneten Zusatz- und/oder Hilfsstoffen in eine geeignete Darreichungsform bringt.

8. Verfahren nach Anspruch 8, dadurch, gekennzeichnet, daß man die Insulinderivate der Formel II (a/b) und/oder deren physiologisch verträgliche Salze in gelöster, amorpher und/oder kristalliner - vorzugsweiser in amorpher und/oder kristalliner - Form einsetzt.

9. Verfahren nach Anspruch 7 und 8, dadurch gekennzeichnet, daß man mindestens eine der folgenden - unter die Formel II (a/b) fallenden - Insulinderivate einsetzt:
A0-Arg-Des-B30-Humaninsulin,
A0-Arg-Humaninsulin,
A0-Arg-B31-Arg-OH-Humaninsulin und
A0-Arg-B31-Arg-B32-Arg-OH-Humaninsulin
sowie deren physiologisch verträglichen Salze.

10. Verfahren nach einem oder mehreren der Ansprüche 7 bis 9, dadurch gekennzeichnet, daß man die pharmakologische Zubereitung als Injektionslösung bzw. -suspension mit einem pH-Wert zwischen etwa 3,0 und 9,0, vorzugsweise etwa 5,0 und 8,5, herstellt.

## Claims (Claims for the following Contracting State(s): DE, GB, FR, IT, NL, SE, LI, CH, BE, AT, LU)

1. An essentially purified insulin derivative of the formula II 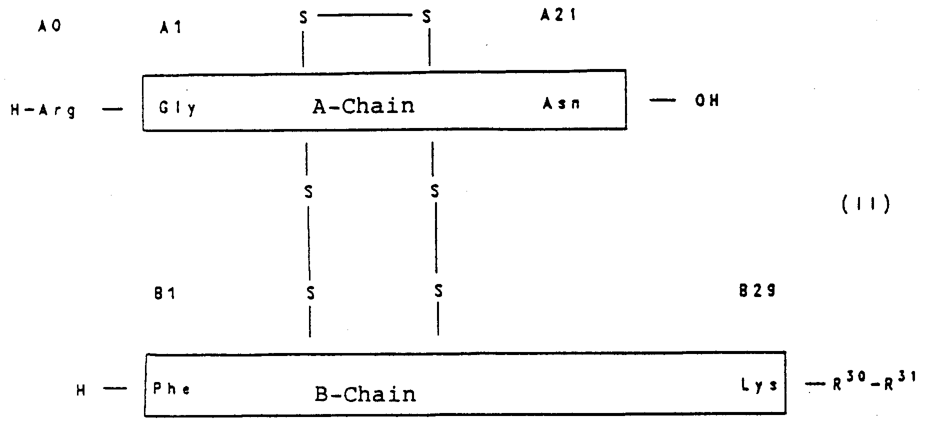 in which
a)
R³⁰ and R³¹ together are OH or
b)
R³⁰ is residue of a neutral, genetically encodable L-amino acid and
R³¹ is OH or 1 to 3 L-amino acids,
and physiologically tolerated salts of the insulin derivative of the formula II, with the exception of the cases in which simultaneously R³⁰ is Ala and R³¹ is OH and the A and B chains are the sequences of beef insulin.

2. An insulin derivative and the physiologically tolerated salts thereof as claimed in claim 1, wherein in formula II R³⁰ + R³¹ = OH.

3. An insulin derivative and the physiologically tolerated salts thereof as claimed in claim 1, wherein for case b) in formula II R³⁰ = radical of Ala, Thr or Ser, in particular of Thr, and R³¹ = OH, Arg-OH or Arg-Arg-OH.

4. An insulin derivative and the physiologically tolerated salts thereof as claimed in one or more of claims 1 to 3, wherein the A chain and the B chain (B1-B29) in formula II are the sequences of human, pork or beef insulin, preferably of human or pork insulin.

5. An insulin derivative as claimed in one or more of claims 1 to 4, which has an isoelectric point between 5.5 and 9.0.

6. A process for the preparation of an insulin derivative of the formula II as defined in one or more of claims 1 to 5, which comprises contacting an insulin product of the formula III or IV 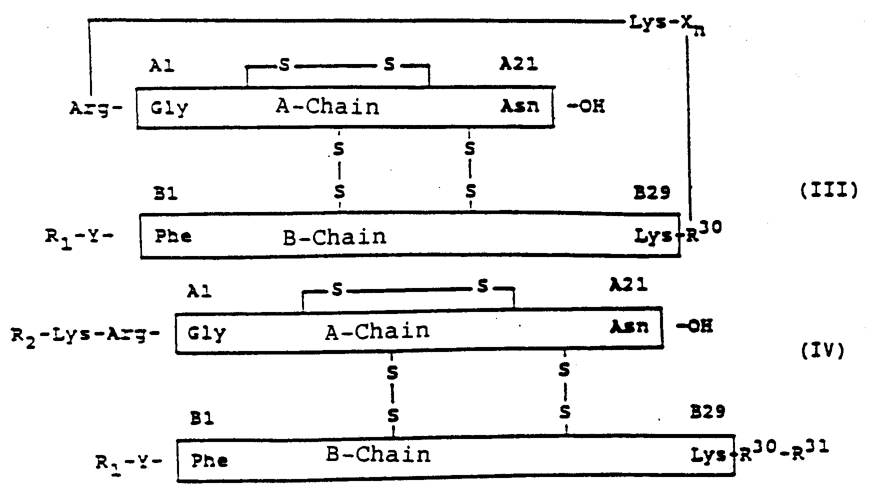 in which
X = residues of identical or different genetically encodable L-amino acids,
Y = Lys or Arg,
n = 0 or integer from 1 - 60,
R₁ and R₂ = OH or
- optionally derivatized - residues of a natural amino acid, or
- optionally derivatized - peptide residues composed of 1-90, preferably 70-80, natural amino acids,
R³⁰ and R³¹ have the same meaning as in formula II, and the A and B(1-29) chains preferably have the sequences of human, pork or beef insulin, in particular of human or pork insulin, with lysyl endopeptidase, there being cleavage of the bonds at the C-terminal end of the lysyl radicals,
and where appropriate - i.e. when Y = Arg - also being mixed with trypsin or a trypsin-like endopeptidase, there being elimination of the moiety R₁-Y from the B chain, and an A0-Arg-de-B30-insulin derivative of the formula IIa being produced, and/or
b) for the preparation of an insulin derivative of the formula IIb, reacting an A0-Arg-de-B30-insulin derivative of the formula IIa in the presence of lysyl endopeptidase or trypsin or of a trypsin-like endopeptidase with a compound of the formula V
H-R³⁰-R³¹ (V),
in which R³⁰ and R³¹ have the meanings specified for formula IIb,
and in which free COOH, OH, SH, NH₂, guanidino and/or imidazole functionalities which are present can be in a form protected in a manner known per se, and subsequently eliminating, in a manner known per se, protective groups which are present where appropriate, or
c) reacting an A0-Arg-de-octapeptide (B23-30)-insulin of the formula VI 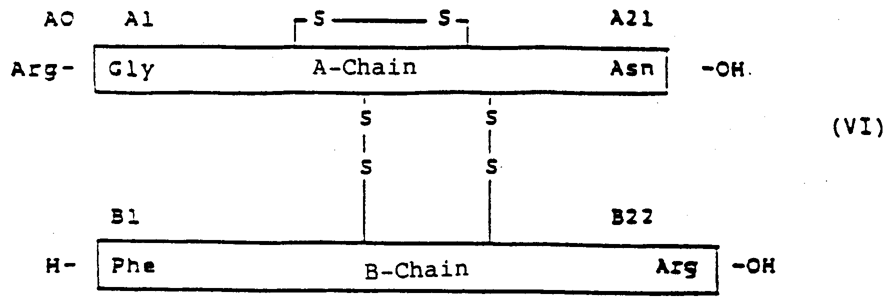 in the presence of trypsin or a trypsin-like endopeptidase with a compound of the formula VII
H-Gly-Phe-Phe-Tyr-Thr-Pro-Lys-R³⁰-R³¹ (VII)
in which R³⁰ and R³¹ have the same meaning as in formula II (a/b),
and in which free COOH, OH, SH, NH₂, guanidino and/or imidazole functionalities which are present can be in a form protected in a manner known per se, and subsequently eliminating, in a manner known per se, protective groups which are present where appropriate, or
d) reacting insulins or derivatives thereof of the formula II' 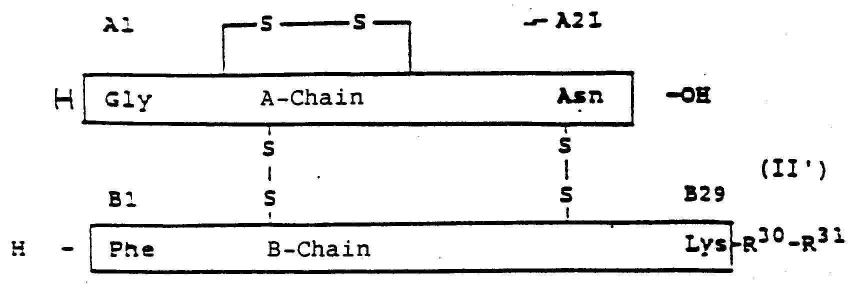 in which R³⁰ and R³¹ have the same meaning as in formula II (a/b),
and in which reactive amino groups - with the exception of the amino group on Al-Gly - are in a form protected in a known manner,
with arginine whose amino groups are likewise protected in a known manner and whose carboxyl group is
in activated form where appropriate, and subsequently eliminating, in a known manner, the protective groups which are present.

7. The use of an insulin derivative and the physiologically tolerated salts thereof as claimed in one or more of claims 1 to 5 as active substances for pharmaceutical formulations for the treatment of diabetes mellitus.

8. A pharmaceutical formulation which contains at least one insulin derivative of the formula II and/or at least one of the physiologically tolerated salts thereof as defined in one or more of claims 1 to 5 in dissolved, amorphous and/or crystalline - preferably in amorphous and/or crystalline - form.

9. A pharmaceutical formulation as claimed in claim 8, which contains at least one of the following insulin derivatives covered by the formula II:
A0-Arg-de-B30-human insulin,
A0-Arg-human insulin,
A0-Arg-B31-Arg-OH-human insulin and
A0-Arg-B31-Arg-B32-Arg-OH-human insulin
and the physiologically tolerated salts thereof.

10. A pharmaceutical formulation as claimed in claim 8 or 9 as solution or suspension for injection having a pH of between about 3.0 and 9.0, preferably about 5.0 and 8.5.

11. A process for the preparation of a pharmaceutical formulation as claimed in one or more of claims 8 to 10, which comprises converting at least one insulin derivative of the formula II and/or at least one of the physiologically tolerated salts thereof, where appropriate together with other modified and/or unmodified insulins or derivatives thereof, with a physiologically acceptable vehicle and, where appropriate, with suitable additives and/or auxiliaries, into a suitable dosage form.

## Claims (Claims for the following Contracting State(s): ES, GR)

1. A process for the preparation of an essentially purified insulin derivative of the formula II 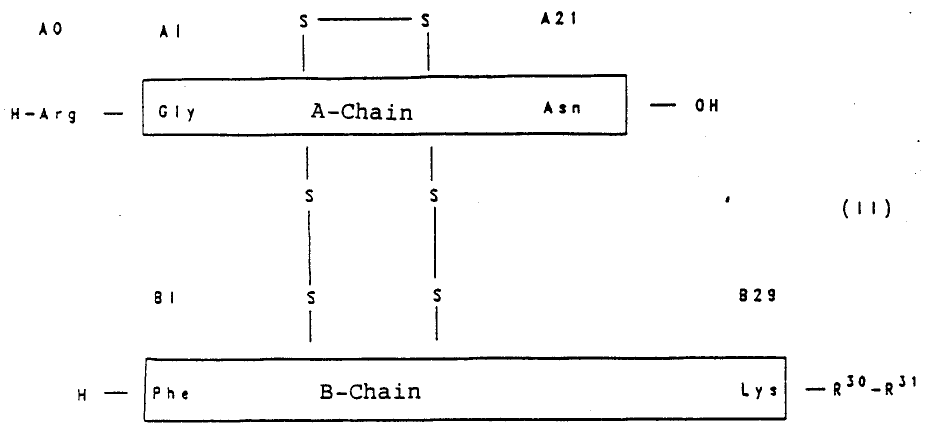 in which
a)
R³⁰ and R³¹ together are OH or
b)
R³⁰ is residue of a neutral, genetically encodable L-amino acid and
R³¹ is OH or 1 to 3 L-amino acids,
and physiologically tolerated salts of the insulin derivative of the formula II, with the exception of the cases in which simultaneously R³⁰ is Ala and R³¹ is OH and the A and B chains are the sequences of beef insulin, which comprises
a) contacting an insulin product of the formula III or IV 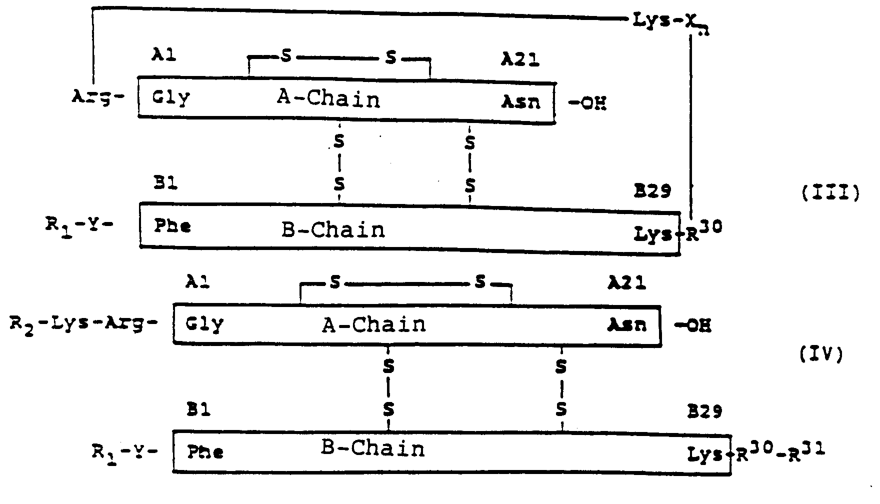 in which
X = residues of identical or different genetically encodable L-amino acids,
Y = Lys or Arg,
n = 0 or integer from 1 - 60,
R₁ and R₂ = OH or
- optionally derivatized - residues of a natural amino acid, or
- optionally derivatized - peptide residues composed of 1-90, preferably 70-80, natural amino acids,
R³⁰ and R³¹ have the same meaning as in formula II, and the A and B(1-29) chains preferably have the sequences of human, pork or beef insulin, in particular of human or pork insulin, with lysyl endopeptidase, there being cleavage of the bonds at the C-terminal end of the lysyl radicals,
and where appropriate - i.e. when Y = Arg - also being mixed with trypsin or a trypsin-like endopeptidase, there being elimination of the moiety R₁-Y from the B chain, and an A0-Arg-de-B30-insulin derivative of the formula IIa being produced, and/or
b) for the preparation of an insulin derivative of the formula IIb, reacting an A0-Arg-de-B30-insulin derivative of the formula IIa in the presence of lysyl endopeptidase or trypsin or of a trypsin-like endopeptidase with a compound of the formula V
H-R-³⁰-R³¹ (V),
in which R³⁰ and R³¹ have the meanings specified for formula IIb,
and in which free COOH, OH, SH, NH₂, guanidino and/or imidazole functionalities which are present can be in a form protected in a manner known per se, and subsequently eliminating, in a manner known per se, protective groups which are present where appropriate, or
c) reacting an A0-Arg-de-octapeptide (B23-30)-insulin of the formula VI 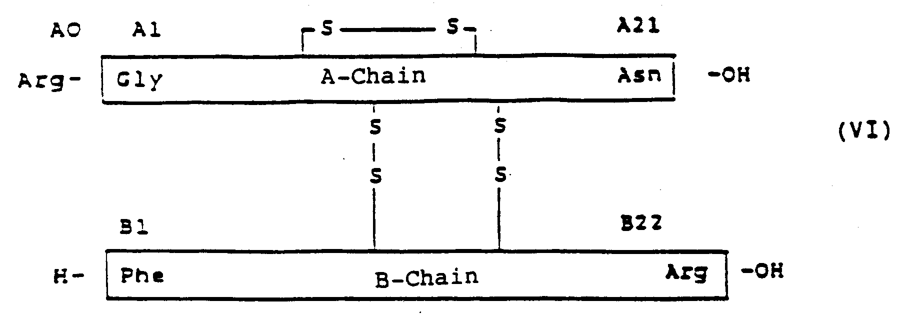 in the presence of trypsin or a trypsin-like endopeptidase with a compound of the formula VII
H-Gly-Phe-Phe-Tyr-Thr-Pro-Lys-R³⁰-R³¹ (VII)
in which R³⁰ and R³¹ have the same meaning as in formula II (a/b),
and in which free COOH, OH, SH, NH₂, guanidino and/or imidazole functionalities which are present can be in a form protected in a manner known per se, and subsequently eliminating, in a manner known per se, protective groups which are present where appropriate, or
d) reacting insulins or derivatives thereof of the formula II' 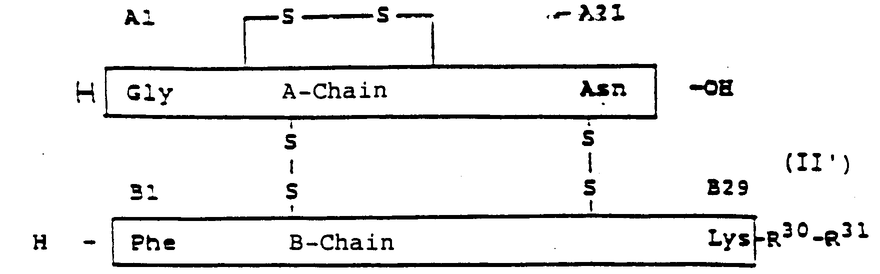 in which R³⁰ and R³¹ have the same meaning as in formula II (a/b),
and in which reactive amino groups - with the exception of the amino group on Al-Gly - are in a form protected in a known manner,
with arginine whose amino groups are likewise protected in a known manner and whose carboxyl group is in activated form where appropriate, and subsequently eliminating, in a known manner, the protective groups which are present,
and optionally converting the end products of all the process variants a-d into their physiologically tolerated salts.

2. The process as claimed in claim 1, wherein insulin derivatives of the formula IIa are prepared, in which R³⁰ + R³¹ together = OH, and are optionally converted into their physiologically tolerated salts.

3. The process as claimed in claim 1, wherein insulin derivatives of the formula IIb are prepared, in which R³⁰ = radical of Ala, Thr or Ser, in particular of Thr, and R³¹ = OH, Arg-OH or Arg-Arg-OH, and are optionally converted into their physiologically tolerated salts.

4. The process as claimed in one or more of claims 1 to 3, wherein compounds of the formula II are prepared, in which the A chain and the B chain (B1-B29) are the sequences of human, pork or beef insulin, preferably of human or pork insulin, and are optionally converted into their physiologically tolerated salts.

5. The process as claimed in one or more of claims 1 to 4, wherein compounds of the formula II are prepared which have an isoelectric point between 5.5 and 9.0.

6. The use of an insulin derivative of the formula II (a/b) as defined in claim 1 and the physiologically tolerated salts thereof as active substances for pharmaceutical formulations for the treatment of Diabetes mellitus.

7. A process for the preparation of a pharmaceutical formulation, which comprises converting at least one insulin derivative of the formula II (a/b) as defined in claim 1 and/or at least one of the physiologically tolerated salts thereof, where appropriate together with other modified and/or unmodified insulins or derivatives thereof, with a physiologically acceptable vehicle and, where appropriate, with suitable additives and/or auxiliaries, into a suitable dosage form.

8. The process as claimed in claim 7, wherein the insulin derivatives of the formula II (a/b) and/or the physiologically tolerated salts thereof are employed in dissolved, amorphous and/or crystalline - preferably in amorphous and/or crystalline - form.

9. The process as claimed in claim 7 and 8, wherein at least one of the following insulin derivatives covered by the formula II (a/b):
A0-Arg-de-B30-human insulin,
A0-Arg-human insulin,
A0-Arg-B31-Arg-OH-human insulin and
A0-Arg-B31-Arg-B32-Arg-OH-human insulin
and the physiologically tolerated salts thereof, is employed.

10. The process as claimed in one or more of claims 7 to 9, wherein the pharmacological formulation is prepared as solution or suspension for injection having a pH of between about 3.0 and 9.0, preferably about 5.0 and 8.5.

## Revendications (Revendications pour l'(les) Etat(s) contractant(s) suivant(s): DE, GB, FR, IT, NL, SE, LI, CH, BE, AT, LU)

1. Dérivé d'insuline pratiquement purifié de formule II 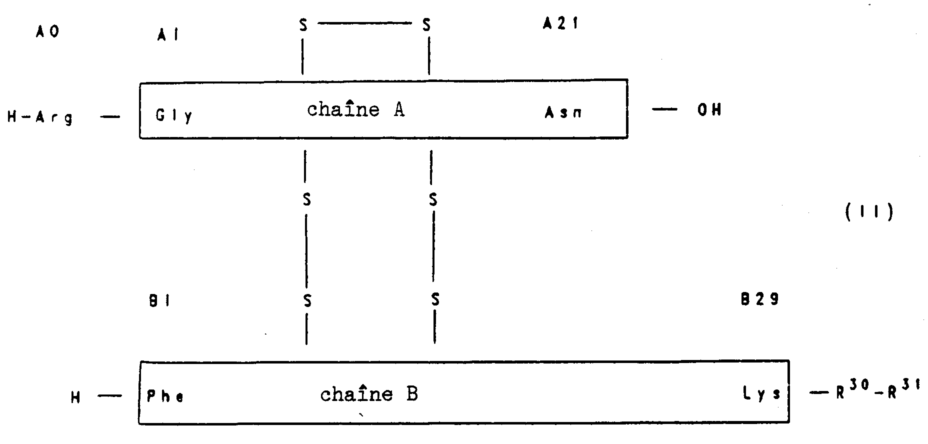 dans laquelle
a)
R³⁰ et R³¹ représentent ensemble OH ou
b)
R³⁰ représente le reste d'un L-aminoacide neutre, génétiquement codable, et
R³¹ représente OH ou 1 à 3 L-aminoacides,
et sels physiologiquement acceptables du dérivé d'insuline de formule II, à l'exception du cas dans lequel simultanément R³⁰ est Ala et R³¹ est OH, ainsi que les chaînes A et B sont les séquences de l'insuline bovine.

2. Dérivés d'insuline et leurs sels physiologiquement acceptables selon la revendication 1, caractérisés en ce que, dans la formule II R³⁰ + R³¹ = OH.

3. Dérivés d'insuline et leurs sels physiologiquement acceptables selon la revendication 1, caractérisés en ce que, dans la formule II, pour le cas b), R³⁰ représente le reste de Ala, Thr ou Ser, en particulier de Thr, et R³¹ = OH, Arg-OH ou Arg-Arg-OH.

4. Dérives d'insuline et leurs sels physiologiquement acceptables selon une ou plusieurs des revendications 1 à 3, caractérisés en ce que, dans la formule II, la chaîne A et la chaîne B (B1 - B29) sont les séquences de l'insuline humaine, porcine ou bovine, de préférence de l'insuline humaine ou porcine.

5. Dérivés d'insuline et leurs sels physiologiquement acceptables selon une ou plusieurs des revendications 1 à 4, caractérisés par un point isoélectrique compris entre 5,5 et 9,0.

6. Procédé pour la préparation de dérivés d'insuline de formule II selon la définition dans une ou plusieurs des revendications 1 à 5, caractérisé en ce que
a) on met en contact avec de la lysylendopeptidase un produit de type insuline de formule III ou IV 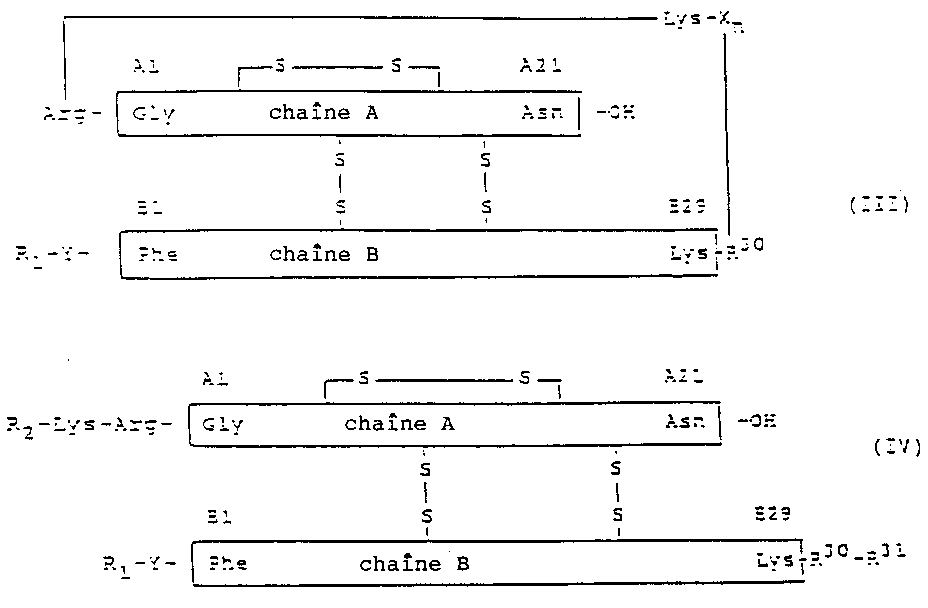 formules dans lesquelles
X représente des restes de L-aminoacides identiques ou différents, génétiquement codables,
Y = Lys ou Arg,
n = 0 ou un nombre entier allant de 1 à 60,
R₁ et R₂ = OH ou
des restes - éventuellement transformés en dérivés - d'un aminoacide naturel, ou des restes peptidiques - éventuellement transformés en dérivés - constitués de 1 à 90, de préférence de 70 à 80 aminoacides naturels,
R³⁰ et R³¹ ont les mêmes significations que dans la formule II, et les chaînes A et B (1 - 29) présentant de préférence les séquences de l'insuline humaine, porcine ou bovine, en particulier de l'insuline humaine ou porcine,
ce par quoi les liaisons à l'extrémité C-terminale des restes lysyle sont coupées,
et éventuellement - c'est-à-dire lorsque Y = Arg - on ajoute encore de la trypsine ou une endopeptidase de type trypsine, ce par quoi la queue R₁-Y de la chaîne a est coupée, et il en résulte un dérivé AO-Arg-des-B30-insuline de formule IIa, et/ou en ce que
b) pour la préparation d'un dérivé d'insuline de formule IIb, on fait réagir un dérivé AO-Arg-des-B30-insuline de formule IIa, en présence de lysylendopeptidase ou de trypsine, ou d'une endopeptidase de type trypsine, avec un composé de formule V
H-R³⁰-R³¹ (V),
dans laquelle R³⁰ et R³¹ ont les significations indiquées à propos de la formule IIb,
et dans laquelle des fonctions COOH, OH, SH, NH₂, guanidino et/ou imidazole libres présentes peuvent se trouver protégées d'une façon connue en soi,
et ensuite on élimine, d'une façon connue en soi, des groupes protecteurs éventuellement présents, ou en ce que
c) on fait réagir une AO-Arg-des-octapeptide(B23-30)-insuline de formule VI 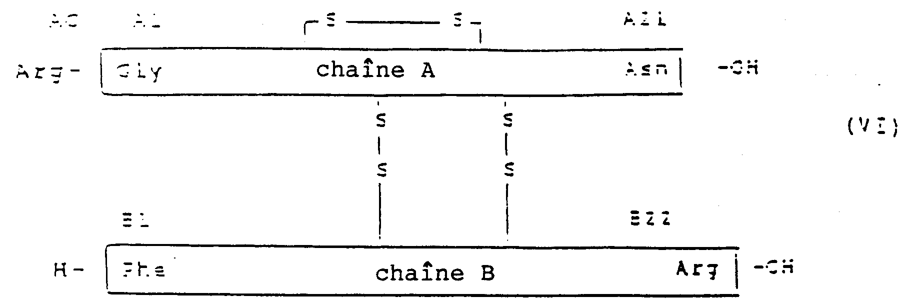 en présence de trypsine ou d'une endopeptidase de type trypsine, avec un composé de formule VII
H-Gly-Phe-Phe-Tyr-Thr-Pro-Lys-R³⁰-R³¹ (VII)
dans laquelle R³⁰ et R³¹ ont les mêmes significations que dans la formule II (a/b),
et dans laquelle des fonctions COOH, OH, SH, NH₂, guanidino et/ou imidazole libres présentes peuvent se trouver protégées, d'une façon connue en soi, et ensuite on élimine d'une façon connue en soi des groupes protecteurs éventuellement présents, ou en ce que
d) on fait réagir un(des) dérivé(s) d'insuline de formule II' 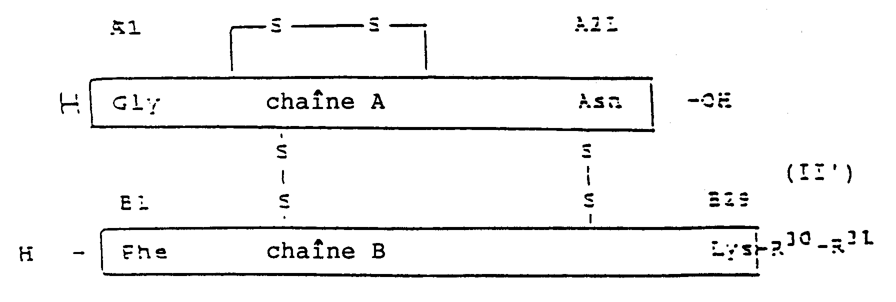 dans laquelle R³⁰ et R³¹ ont les mêmes significations que dans la formule II (a/b), et dans laquelle des groupes amino réactifs - à l'exception du groupe amino sur Al-Gly - se trouvent protégés de façon connue,
avec de l'arginine dont les groupes amino sont également protégés de façon connue, et dont le groupe carboxy se trouve éventuellement sous forme activée, et ensuite on élimine de façon connue les groupes protecteurs présents.

7. Utilisation des dérivés d'insuline et de leurs sels physiologiquement acceptables selon une ou plusieurs des revendications 1 à 5, en tant que substances actives pour des compositions pharmaceutiques destinées au traitement du diabète sucré.

8. Composition pharmaceutique, caractérisée par une teneur en au moins un dérivé d'insuline de formule II et/ou au moins un de ses sels physiologiquement acceptables selon la définition dans une ou plusieurs des revendications 1 à 5, sous forme dissoute, amorphe et/ou sous forme cristalline, de préférence sous forme amorphe et/ou sous forme cristalline.

9. Composition pharmaceutique selon la revendication 8, caractérisée par une teneur en au moins l'un des dérivés d'insuline - rentrant dans le cadre de la formule II suivants:
AO-Arg-des-B30-insuline humaine,
AO-Arg-insuline humaine,
AO-Arg-B31-Arg-OH-insuline humaine et
AO-Arg-B31-Arg-B32-Arg-OH-insuline humaine,
ainsi que leurs sels physiologiquement acceptables.

10. Composition pharmaceutique selon la revendication 8 ou 9, sous forme de solution ou suspension injectable ayant un pH compris entre environ 3,0 et 9,0, de préférence entre environ 5,0 et 8,5.

11. Procédé pour la préparation d'une composition pharmaceutique selon une ou plusieurs des revendications 8 à 10, caractérisé en ce que l'on met sous une forme d'administration appropriée au moins un dérivé d'insuline de formule II et/ou au moins un de ses sels physiologiquement acceptables, éventuellement avec un autre(d'autres) dérivé(s) d'insuline modifié(s) et/ou non modifié(s), avec un véhicule physiologiquement acceptable ainsi qu'éventuellement des additifs et/ou adjuvants appropriés.

## Revendications (Revendications pour l'(les) Etat(s) contractant(s) suivant(s): ES, GR)

1. Procédé pour la préparation d'un dérivé d'insuline pratiquement purifié de formule II 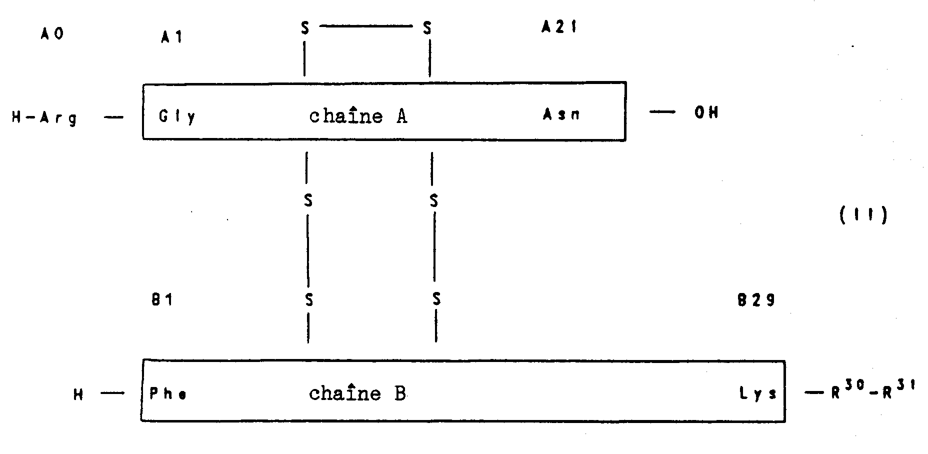 dans laquelle
a)
R³⁰ et R³¹ représentent ensemble OH ou
b)
R³⁰ représente le reste d'un L-aminoacide neutre, génétiquement codable, et
R³¹ représente OH ou 1 à 3 L-aminoacides, ainsi que de sels physiologiquement acceptables du dérivé
d'insuline de formule II, à l'exception du cas dans lequel simultanément R³⁰ est Ala et R³¹ est OH, ainsi que les chaînes A et B sont les séquences de l'insuline bovine, caractérisé en ce que
a) on met en contact avec de la lysylendopeptidase un produit de type insuline de formule III ou IV 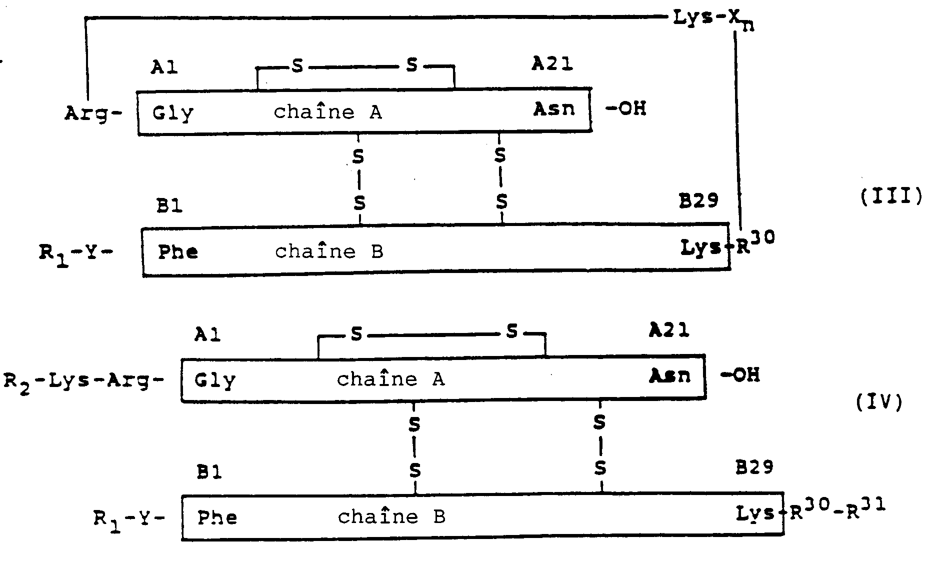 formules dans lesquelles
X représente des restes de L-aminoacides identiques ou différents, génétiquement codables,
Y = Lys ou Arg,
n = 0 ou un nombre entier allant de 1 à 60,
R₁ et R₂ = OH ou des restes - éventuellement transformés en dérivés - d'un aminoacide naturel, ou des restes peptidiques - éventuellement transformés en dérivés - constitués de 1 à 90, de préférence de 70 à 80 aminoacides naturels,
R³⁰ et R³¹ ont les mêmes significations que dans la formule II, et les chaînes A et B (1 - 29) comportent de préférence les séquences de l'insuline humaine, porcine ou bovine, en particulier de l'insuline humaine ou porcine,
ce par quoi les liaisons à l'extrémité C-terminale des restes lysyle sont coupées, et éventuellement - c'est-à-dire lorsque Y = Arg - on ajoute encore de la trypsine ou une endopeptidase de type trypsine, ce par quoi la queue R₁-Y de la chaîne B est coupée, et il en résulte un dérivé AO-Arg-des-B30-insuline de formule IIa, et/ou en ce que
b) pour la préparation d'un dérivé d'insuline de formule IIb, on fait réagir un dérivé AO-Arg-des-B30-insuline de formule IIa, en présence de lysylendopeptidase ou de trypsine, ou d'une endopeptidase de type trypsine, avec un composé de formule V
H-R³⁰-R³¹ (V),
dans laquelle R³⁰ et R³¹ ont les significations indiquées à propos de la formule IIb,
et dans laquelle des fonctions COOH, OH, SH, NH₂, guanidino et/ou imidazole libres présentes peuvent se trouver protégées d'une façon connue en soi,
et ensuite on élimine, d'une façon connue en soi, des groupes protecteurs éventuellement présents, ou en ce que
c) on fait réagir une AO-Arg-des-octapeptide(B23-30)-insuline de formule VI 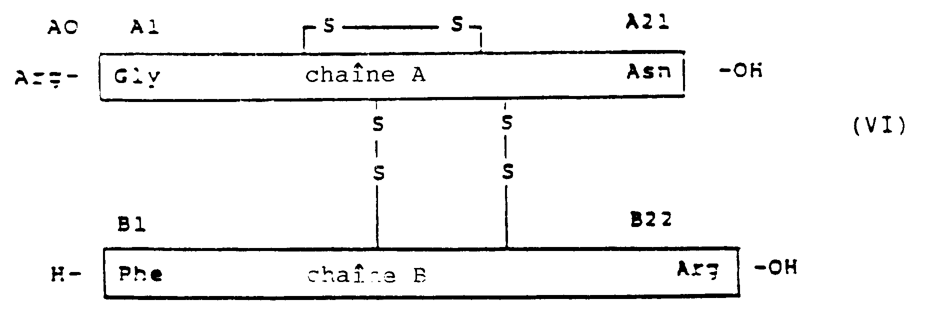 en présence de trypsine ou d'une endopeptidase de type trypsine, avec un composé de formule VII
H-Gly-Phe-Phe-Tyr-Thr-Pro-Lys-R³⁰-R³¹ (VII)
dans laquelle R³⁰ et R³¹ ont les mêmes significations que dans la formule II (a/b),
et dans laquelle des fonctions COOH, OH, SH, NH₂, guanidino et/ou imidazole libres présentes peuvent se trouver protégées, d'une façon connue en soi, et ensuite on élimine d'une façon connue en soi des groupes protecteurs éventuellement présents, ou en ce que
d) on fait réagir un(des) dérivé(s) d'insuline de formule II' 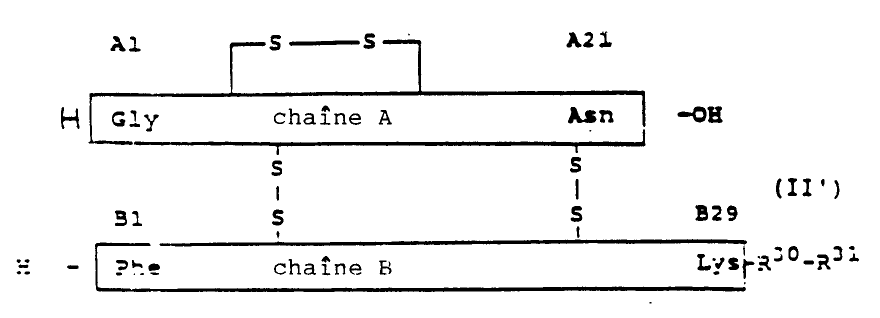 dans laquelle R³⁰ et R³¹ ont les mêmes significations que dans la formule II (a/b),
et dans laquelle des groupes amino réactifs - à l'exception du groupe amino sur Al-Gly - se trouvent protégés de façon connue,
avec de l'arginine dont les groupes amino sont également protégés de façon connue, et dont le groupe carboxy se trouve éventuellement sous forme activée, et ensuite on élimine de façon connue les groupes protecteurs présents,
et en ce que éventuellement on convertit en leurs sels physiologiquement acceptables les produits finals de toutes les variantes a-d du procédé.

2. Procédé selon la revendication 1, caractérisé en ce que l'on prépare des dérivés d'insuline de formule IIa dans lesquels R³⁰ + R³¹ représentent ensemble OH, et éventuellement on les convertit en leurs sels physiologiquement acceptables.

3. Procédé selon la revendication 1, caractérisé en ce que l'on prépare des dérivés d'insuline de formule IIb, dans lesquels R³⁰ représente un reste de Ala, Thr ou Ser, en particulier de Thr, et R³¹ = OH, Arg-OH ou Arg-Arg-OH, et éventuellement on les convertit en leurs sels physiologiquement acceptables.

4. Procédé selon une ou plusieurs des revendications 1 à 3, caractérisé en ce que l'on prépare des composés de formule II dans lesquels la chaîne A et la chaîne B (B1 - B29) sont les séquences de l'insuline humaine, porcine ou bovine, de préférence de l'insuline humaine ou porcine, et éventuellement on les convertit en leurs sels physiologiquement acceptables.

5. Procédé selon une ou plusieurs des revendications 1 à 4, caractérisé en ce que l'on prépare des composés de formule II ayant un point isoélectrique compris entre 5,5 et 9,0.

6. Utilisation des dérivés d'insuline de formule II (a/b) selon la définition dans la revendication 1, et de leurs sels physiologiquement acceptables, en tant que substances actives pour des compositions pharmaceutiques destinées au traitement du diabète sucré.

7. Procédé pour la préparation d'une composition pharmaceutique, caractérisé en ce que l'on met sous une forme d'administration appropriée au moins un dérivé d'insuline de formule II (a/b) selon la définition dans la revendication 1 et/ou au moins l'un de ses sels physiologiquement acceptables, eventuellement avec un autre(d'autres) dérivé(s) d'insuline modifié(s) et/ou non modifié(s), avec un véhicule physiologiquement acceptable ainsi qu'éventuellement des additifs et/ou adjuvants appropriés.

8. Procédé selon la revendication 7, caractérisé en ce que l'on utilise les dérivés d'insuline de formule II (a/b) et/ou leurs sels physiologiquement acceptables, sous forme dissoute, amorphe et/ou cristalline, de préférence sous forme amorphe et/ou sous forme cristalline.

9. Procédé selon la revendication 7 ou 8, caractérisé en ce que l'on utilise au moins l'un des dérivés d'insuline - rentrant dans le cadre de la formule II (a/b) - suivants:
AO-Arg-des-B30-insuline humaine,
AO-Arg-insuline humaine,
AO-Arg-B31-Arg-OH-insuline humaine et
AO-Arg-B31-Arg-B32-Arg-OH-insuline humaine,
ainsi que leurs sels physiologiquement acceptables.

10. Procédé selon une ou plusieurs des revendications 7 à 9, caractérisé en ce que l'on prépare la composition pharmaceutique sous forme d'une solution ou suspension injectable ayant un pH compris entre environ 3,0 et 9,0, de préférence entre environ 5,0 et 8,5.
